# EUROPEAN PATENT SPECIFICATION

(11) **EP 1 842 534 B1**
(45) Date of publication and mention of the grant of the patent: **14.12.2011**
(21) Application number: 06252598.5
(22) Date of filing: 18.05.2006
(51) Int. Cl.: A61K 9/26, A61K 9/52, A61K 31/138

(54) **Metoprolol succinate extended release tablets and methods for their preparation**
Metoprololsuccinat Tabletten mit verlängerter Freisetzung und Verfahren für deren Herstellung
Comprimés de succinate de metoprolol à libération étendue et procédés de fabrication

(30) Priority: 24.02.2006 US 776706 P
(43) Date of publication of application: 10.10.2007
(73) Proprietor: Teva Pharmaceutical Industries Ltd., 49131 Petah Tiqva (IL)
(72) Inventor: Gold, Tomer, Ra'anana 43315 (IL); Shterman, Nava, Petach Tikva 49214 (IL)
(74) Representative: Wong, Kit Yee

(56) References cited:
- WO-A-96/01621
- US-A- 4 871 549
- US-A- 5 246 714
- US-A1- 2003 185 887
- US-A1- 2005 008 701

## Description

### FIELD OF THE INVENTION

The present invention relates to an extended release pharmaceutical composition of a beta blocker such as metoprolol succinate, as the active ingredient, and methods of preparing the extended release pharmaceutical composition.

### BACKGROUND OF THE INVENTION

Metoprolol succinate is a beta₁-selective (cardioselective) adrenoceptor blocking agent, for oral administration, available as extended release tablets. In the prior art, metoprolol succinate has apparently been formulated to provide a controlled and predictable release of metoprolol for once-daily administration. The tablets reportedly comprise a multiple unit system containing metoprolol succinate in a multitude of controlled release pellets. Each pellet supposedly acts as a separate drug delivery unit and is designed to deliver metoprolol continuously over the dosage interval. The tablets contain 23.75, 47.5, 95 and 190 mg of metoprolol succinate equivalent to 25, 50, 100 and 200 mg of metoprolol tartrate, respectively. Its chemical name is (±)1-(isopropylamino)-3-[p-(2-methoxyethyl) phenoxy]-2-propanol succinate (2:1) (salt). Its structural formula is apparently:

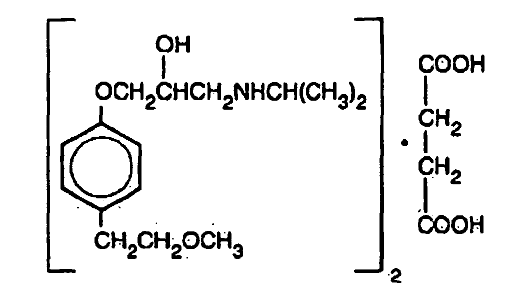

An extended release tablet of Metoprolol succinate is currently being marketed as TOPROL XL^{®}, as a beta₁-selective adrenoceptor blocking agent. According to the prescribing information TOPROL XL^{®} is indicated for the treatment of hypertension, the long term treatment of angina pectoris, and the treatment of stable symptomatic (NYHA Class II or III) heart failure of ischemic, hypertensive or cardiomyopathic origin. In general, commercially available metoprolol succinate E.R. tablets contain in addition to the active pharmaceutical ingredient the following inactive ingredients: silicon dioxide, cellulose compounds, sodium stearyl fumarate, polyethylene glycol, titanium dioxide, and paraffin.

In US patent 4,927,640 a composition and method to produce such composition is described which apparently requires beads that are selected from the group consisting of glass and silicon dioxide and which are insoluble in water, physiological fluids and liquids commonly used for intravenous infusion. These beads are covered with one or more pharmaceutically active compounds and a release controlling polymeric membrane covering the active layer.

US patent 4,957,745 apparently describes more specifically a controlled release preparation comprising a plurality of beads having a soluble component comprising at least 95% weight/weight of a metoprolol salt which salt has a solubility of less than 600mg/ml in water at 25°C. The controlling polymeric membrane is described apparently as consisting essentially of ethylcellulose, or a mixture of ethylcellulose and hydroxypropyl-methylcellulose. In the examples in US patent 4,957,745 the metoprolol salt is apparently applied on silicon dioxide beads, which beads are sized between 150µm - 250µm.

Both US patent 4,927,640 and US patent 4,957,745 apparently describe a method for producing coated beads and tablets. The beads are understood to be covered with a metoprolol salt layer. This metoprolol salt layer is applied onto the beads after mixing the salt with methylene chloride and ethanol. An additional rate controlling layer is then applied after using methylene chloride and isopropyl alcohol as solvents. Methylene chloride however is described in the "GUIDANCE FOR INDUSTRY, Q3C - Tables and List", published by the Food and Drug Administration as a solvent with "inherent toxicity". Further, the beads, as described above, can be compressed into tablets. The additives described are pharmaceutical acceptable excipients for preparation in a wet granulation process.

US patent 5,246,714 also apparently describes a composition and method for the preparation of beads containing a pharmaceutically active ingredient compressed into tablets. Again, the use of toxic solvents, the use of additives to produce a tablet mass with the beads for preparation in a wet granulation process are described.

US 2005/0008701 relates to a controlled release pellet comprising: (a) an inert core that is water soluble or water swellable; (b) a drug layer applied to the inert core comprising: (i) a beta, adrenergic blocking agent, and (ii) optionally, a binder, and (c) a controlled release coating surrounding the drug layer.

The present invention relates to an extended release composition, comprising a plurality of pellets, each comprising a beta blocker agent and pharmaceutical acceptable excipients. The advantage of the present invention is that it provides a composition and a method of preparation thereof, which does not incorporate the use of inherent toxic solvents. Moreover, the tablets are prepared using a direct compression process, instead of using a wet granulation process, while still producing a uniform product. The production process is, hence, shortened, and machinery such as a high speed high shear mixer and a milling apparatus are not required. The use of commercially available excipients such as sugar spheres further allows for the reduction of production costs and time.

### SUMMARY OF THE INVENTION

The present invention provides a suitable extended release composition comprising metoprolol and various excipients. In particular there is provided an extended release pharmaceutical composition that can be prepared comprising an inert core, an active pharmaceutical ingredient layer, and a controlled/extended release coating layer, without using inherently toxic solvents.

The present invention provides a pharmaceutical composition for extended release comprising pellets coated with an active pharmaceutical ingredient wherein each coated pellet comprises
a) an inert core comprising at least about 50% (w/w) of soluble substance;
b) a drug layer comprising metropolol or a pharmaceutically acceptable salt thereof, which layer covers the inert core; and
c) a controlled release layer thereon;
and wherein the inert core comprises an initial core and a sub-coat thereon and wherein the sub-coat layer comprises at least one hydrophobic film coating polymer.

In addition, the pharmaceutical composition preferably comprises a plurality of pellets. Further, the pharmaceutical composition is preferably prepared without the use of inherently toxic solvents.

The present invention also provides a method of preparing a pharmaceutical composition comprising pellets coated with an active pharmaceutical ingredient comprising the steps of
a) providing an inert core comprising at least 50% (w/w) of soluble substance;
b) applying a drug layer comprising metoprolol or a pharmaceutically acceptable salt thereof onto the inert core forming a drug coated pellet;
c) coating the drug coated pellet with a controlled release layer forming a coated pellet;
and wherein the inert core comprises an initial core and a sub-coat thereon and wherein the sub-coat layer comprises at least one hydrophobic film coating polymer.

Preferably, the method further comprises the step of coating an initial core/sphere with a sub-coat forming the inert core before applying a drug layer onto the inert core. Further, the method of preparing the pharmaceutical composition preferably does not use any inherently toxic solvents, Moreover, the method may further comprise the steps of d) mixing the coated pellets with one or more excipients to form a final blend; and e) tableting the final blend. Preferably, the final blend is tableted using a direct compression method.

The present invention also provides the use of a pharmaceutical composition as described above for the manufacture of a medicament for the treatment of a disorder susceptible to treatment with a beta₁-selective adrenoceptor blocking agent.

Preferably, the composition is used in the treatment of patients suffering from hypertension, angina pectoris or stable symptomatic (NYHA Class II or III) heart failure of ischemic, hypertensive or cardiomyopathic origin.

### BRIEF DESCRIPTION OF THE FIGURES

- Figure 1. :: Shows an in vitro dissolution profile for two formulations of pellets comprising substantially different amounts of inert core.
- Figure 2. :: Shows an in vitro dissolution profile of two formulations of pellets comprising different ratios of hydrophobic to hydrophilic plasticizers in the controlled release layer.
- Figure 3. :: Shows an in vitro dissolution profile for three formulations of pellets wherein formulation K-35180/B2 has no sub-coating on the sugar spheres and formulations K-35222/C2 and K-35104/E2 have different amounts of sub-coatings.
- Figure 4. :: Shows an in vitro dissolution profile of a tablet formulation comprising pellets of the invention according to the method of example 6.

### DETAILED DESCRIPTION OF THE INVENTION

The present invention provides an extended release tablet comprising metoprolol succinate pellets and pharmaceutically acceptable excipients such as for example binders, film coating polymers, plasticizers, starch, glidants, and disintegrants.

As used herein the term initial core refers to a pharmaceutically acceptable core for use in pharmaceutical formulations which core is inert and which is commercially available and has not been modified by for example a treatment applying a sub-coat onto the core. Further, as used herein the term inert core refers to a pharmaceutically acceptable core for use in pharmaceutical formulations which is inert, is commercially available and which may be modified by for example a treatment, as in the present invention, applying a sub-coat onto the core. In addition, as used herein the term soluble substances refers to substances which may completely dissolve in an aqueous environment such as the gastrointestinal tract of a patient.

In one embodiment of the present invention there is provided a pharmaceutical composition for extended release comprising pellets coated with an active pharmaceutical ingredient wherein each coated pellet comprises
a) an inert core comprising at least 50% (w/w) of soluble substance;
b) a drug layer comprising the active pharmaceutical ingredient, which layer covers the inert core; and
c) a controlled release layer thereon.
A sub-coat layer covers an initial core/sphere forming the inert core. In addition, the pharmaceutical composition of the present invention preferably comprises a plurality of coated pellets, coated with a first layer comprising the active pharmaceutical ingredient (API), drug, and a second controlled release layer. The API (drug) is metoprolol or one of its pharmaceutical acceptable salts, each pellet thus comprising an inert core, a drug layer and a rate controlling film coating. Metoprolol succinate is the most preferred API. Moreover, the pharmaceutical composition is preferably prepared without the use of inherently toxic solvents. The drug layer is preferably applied as a suspension of finely divided solid API rather than a solution.

In another embodiment there is provided a pharmaceutical composition of the present invention wherein the release rate of drug from the pellets part of the pharmaceutical composition comprising a tabletted or encapsulated composition of a multitude of pellets is controlled by the amount or the percentage of the initial core/spheres of the pellets. Preferably, the amount of initial core is from about 15% to about 30% by weight of the controlled release coated pellets before tableting or capsule filling. More preferably, the amount of initial core is about 22% of the extended release coated pellet before tableting or capsule filling. In addition, the amount of inert core (as a combination of an initial core and sub-coat as described below) is preferably from about 20% to about 35% by weight of the controlled release coated pellets before tableting or capsule filling. More preferably, the amount of inert core is about 27% of the extended release coated pellet before tableting

The inert core is strengthened by applying a sub-coat on the initial core/sphere of the present invention. In pharmaceutical compositions wherein pellets comprising the drug are compressed into tablets the drug pellets are mixed with powder excipients to form a tableting blend. However, the size of the drug coated pellets, often larger than the particle size of the powder excipients, can cause a lack of uniformity of the tableting blend. The preferred uniformity of the tableting blend is such that the average assay of ten samples of the tableting blend each weighing the equivalent of one tablet lies within the range of 90 to 110 percent of the label dose and the relative standard deviation of the individual assays is less than or equal to 5 percent. The size of the drug pellets is therefore preferably small. When layering a large amount of drug on a small initial core a high degree of stress is exerted on the initial core. This stress may cause attrition particularly when the inert core comprises sugar spheres. To provide a higher degree of physical strength of the inert core without changing the dissolution rate of drug coated pellets, a sub-coat is applied on an initial core/sphere. Preferably, the amount of the sub-coat is from about 10% to about 40% of the total weight of the sub-coated inert core, more preferably the amount of sub-coat is from about 15% to about 30% of the total weight of the sub-coated inert core, most preferably the amount of sub-coat is about 16% to about 20% of the total weight of the sub-coated inert core.

In one embodiment there is provided a pharmaceutical composition of the present invention wherein the release rate of drug from the part of the pharmaceutical composition comprising a multitude of pellets is controlled by the ratio of hydrophilic to hydrophobic plasticizers in the controlled release layer. The controlled release layer in the pharmaceutical composition of the present invention preferably comprises a hydrophobic film coating polymer such as for example ethylcellulose or polymethacrylates in combination with at least two plasticizers, at least one hydrophilic and one hydrophobic plasticizer. Preferably, the ratio of hydrophobic to hydrophilic plasticizer in the controlled release layer of the pharmaceutical composition of the present invention is from 3:1 to 1:3, more preferably the ratio is 1:1.

The inert core of each of the pellets in the pharmaceutical composition of the present invention comprises from about 50% to about 100% (per weight) of soluble substance. Preferably the inert core comprises from about 70% to about 90% (per weight) of soluble substances. A preferred initial core of the present invention comprises a sugar sphere. Sugar spheres have been used in the pharmaceutical industry as excipients. Such sugar spheres used in pharmaceutical compositions generally contain not more than 92% of sucrose, calculated on the dried basis, the remainder consisting of maize starch. Commonly sugar spheres with a core size larger than 500µm are used. The core size of the inert cores in the present invention, preferably a sugar sphere, is between about 50µm and about 500µm, preferably between about 100µm and about 400µm, more preferably from about 250µm to about 350µm.

In the present invention the inert core comprises an initial core/sphere that is sub-coated with a layer of a plasticized film coating polymer. This sub-coating of an initial core/sphere provides physical strength to the inert core of the present invention. The film coating polymer comprises a hydrophobic polymer. Suitable film coating polymers can be cellulose derivative polymers or polymethacrylate polymers. Further, hydrophobic polymers or hydrophilic plasticizers, or a combination of several plasticizers can be used to plasticize the film coating polymers. These compounds of the polymeric sub-coat are mixed with solvents prior to their application onto the initial core/sphere. Suitable solvents for use in mixing the polymeric sub-coating compounds are selected from ethanol, isopropyl alcohol, acetone and purified water. For example a mixture of ethanol, acetone and water is preferred for use in mixing a mixture of the preferred sub-coating compounds EthylCellulose (as a film coating polymer), and plasticizers DiButyl Sebacate and Polyethylene Glycol (EC, DBS and PEG).

Preferably, the initial core/sphere is a sugar sphere which is sub coated with a mixture of polymers such as cellulose derivatives e.g. ethylcellulose and triethyl citrate, polyethylene glycol, dibutyl sebacate, and dibutyl phthalate, and wherein the sub-coating layer on the initial core/sphere does not alter the release rate of the drug for the pharmaceutical composition. A preferred sub-coat on the sugar spheres comprises ethyl cellulose as a hydrophobic film coating polymer and a combination of two or more plasticizers, at least one hydrophilic and at least one hydrophobic plasticizer. Suitable plasticizers may include for example polyethylene glycols, citrate esters, dibutyl sebacate, diethyl phthalate, and triacetin. Preferred plasticizers are polyethylene glycol and dibutyl sebacate as the hydrophilic and hydrophobic plasticizers respectively. Preferably, the sub-coat comprises about 75% to about 85% ethyl cellulose, about 10% to about 20% polyethylene glycol and about 3% to about 7% dibutyl sebacate by weight of the sub-coat. More preferably, the sub-coat comprises 80% ethyl cellulose, 15% polyethylene glycol and 5% dibutyl sebacate by weight of the sub-coat.

Metoprolol or its acceptable pharmaceutical salt is applied on the inert core. No use of "Class 2" solvents (as defined by the FDA) is required to apply the active pharmaceutical ingredient (API), drug, onto the inert core forming a drug coated pellet. The FDA defines "Class 2" solvents as having inherent toxicity. The active ingredient is dispersed in water, preferably together with an acceptable binder excipient such as, but not limited to, polyvinyl pyrrolidone, cellulose derivatives polymers, or starch.

Furthermore, as it is an aspect of the instant invention that the drug substance is applied as a dispersion rather than a solution, it is preferred that the drug substance has physical properties that will allow a high yield in preparing drug coated pellets. Therefore, the drug substance has a particle size distribution such that the d(0.9) value is less than about 80µm. Preferably, the d(0.9) value for the particle size distribution of the drug substance is less than about 50µm, more preferably less then about 30µm. As a result, a concentrated dispersion for application can be produced which may shorten the production time. The drug substance or active pharmaceutical ingredient (API) is metoprolol or one of its pharmaceutically acceptable salts. More preferably, the drug substance is metoprolol succinate.

Moreover, the drug coated pellets comprise from about 40% to about 90% (per weight) of the drug layer, preferably from about 50% to about 80% (per weight), more preferably from about 55% to about 75% (per weight).

The last layer applied on the pellets is a layer which controls the release of the active pharmaceutical ingredient. Pellets of the present invention that have been coated with a controlled release layer have a size between about 200µm and about 800µm. Preferably, the controlled release layer coated pellets have a size ranging from about 300µm to about 700µm, more preferably from about 400µm to about 600µm.

In addition, the controlled release layer comprises water soluble and insoluble components. Such components may be film forming polymers and plasticizers. For example, a film comprising a polymeric layer is applied onto the drug coated pellets. The film comprises at least one film coating polymer and can be plasticized with one or more plasticizers. These plasticizers may differ from each other in their degree of solubility (hydrophobicity/hydrophilicity). By changing the ratio between the plasticizers and the film coating polymer, or the ratio between the different plasticizers (if more than one is used), one can control the rate of the release of the drug from the pellets. The controlled release layer in the pharmaceutical composition of the present invention preferably comprises a hydrophobic film coating polymer such as for example ethylcellulose and a combination of at least two plasticizers, at least one hydrophilic and one hydrophobic plasticizer. Preferably, the ratio of hydrophobic to hydrophilic plasticizer in the controlled release layer of the pharmaceutical composition of the present invention is from 3:1 to 1:3, more preferably the ratio is 1: 1.

Furthermore, the controlled release layer comprises at least about 70% water insoluble compounds (per weight of the controlled release layer). Preferably, the controlled release layer comprises at least about 80% and more preferably at least about 90% water insoluble compounds (per weight of the controlled release layer). Suitable water insoluble compounds are for example cellulose derived polymers. These controlled release layer compounds are mixed with solvents prior to their application onto the drug coated pellets. Suitable solvents for use in mixing the controlled release layer compounds are selected from ethanol, isopropyl alcohol, acetone and purified water. A mixture of ethanol, acetone and water is preferred for use in mixing the controlled release layer compounds especially where the controlled release layer compounds are a mixture of ethylcellulose, dibutyl sebacate and polyethylene glycol. Generally, the drug pellets coated with a controlled release layer of the invention comprise a residual amount of such solvent.

In order to compress these pellets, preferably a plurality of pellets, into tablets or filled into capsules, an additional mass should be incorporated forming a final blend. These additives can be granulated in one of the conventional granulation methods. However, the present invention preferably provides a set of additives, for example a powder mixture that can be directly compressed into tablets. Such powder mixture serves as a filler, cushioning, disintegrant, glidant, and lubricant mixture. Furthermore, the ratio of controlled release drug coated pellets to additives in the final (e.g. tableting) blend of the pharmaceutical composition of the present invention is of particular importance to prepare a uniform product e.g. tablets. The preferred uniformity of the product (uniformity of content by assay) e.g. tablets resulting from this final blend is such that the average assay of ten unit doses (e.g tablets) lies within the range of 90 to 110 percent of the label dose and the relative standard deviation of the individual assays for the doses is less than or equal to 6 percent. In fact, a combination of factors such as the use of additives/powder mixtures with a relatively large particle size and a predetermined controlled release drug coated pellet to additive ratio results in a uniform product.

To prepare such uniform product, preferably at least 50% (by weight) of the powder mixture has particle sizes between about 30µm to about 800 µm, preferably from about 80µm to about 600µm, more preferably from about 100µm to about 300µm. More preferably, at least 65% (by weight) of the powder mixture has particle sizes between about 30 µm to about 800µm, preferably from about 80µm to about 600µm, more preferably from about 100µm to about 300µm. Most preferably, at least 80% (by weight) of the powder mixture has particle sizes between about 30µm to about 800µm, preferably from about 80 µm to about 600µm, most preferably from about 100µm to about 300µm.

Furthermore, the amount of controlled release drug coated pellets in the final tableting blend is preferably from about 20% to about 60% (by weight) in order to prepare such uniform product. More preferably, the amount of controlled release drug coated pellet in the final tableting blend is from about 30% to about 50% (by weight), most preferably from about 35% to about 45% (by weight).

Suitable powder mixtures comprise, but are not limited to, mixtures of two or more of the following compounds; Starlac® (a spray-dried compound consisting of 85% alpha-lactose monohydrate and 15% maize starch dry matter available from Meggle), Cellactose® (a spray-dried compound consisting of 75% alpha-lactose monohydrate and 25% cellulose powder dry matter available from Meggle), Parteck® (A Directly Compressible Sorbitol available from Merck KGaA), Crospovidone, Silicon Dioxide, Magnesium Stearate, Talc, Zinc Stearate, Polyoxyethylene Stearate, Stearic Acid, sodium stearyl fumarate and Cellulose derivatives.

Finally, the tablet may be cosmetically coated with commercially available tablet film coating products such as for example Opadry® available from Colorcon.

In one embodiment of the present invention there is provided a pharmaceutical composition for extended release comprising pellets coated with a beta₁ specific adrenoceptor blocking agent wherein each coated pellet comprises
a) an inert core of sugar spheres coated with a plasticized film sub-coat of a hydrophobic film coating polymer plasticized with a hydrophilic and a hydrophobic plasticizer,
b) a drug layer comprising metoprolol or a pharmaceutically acceptable salt thereof and a binder, and
c) a controlled release layer comprising a plasticized film coat of a hydrophobic film coating polymer plasticized with a hydrophilic and a hydrophobic plasticizer, and wherein the pellets are mixed with a final tableting blend comprising a powder mixture of two or more of fillers, disintegrants, glidants and lubricants.

Preferably, this pharmaceutical composition of the present invention comprises:

| **Material** | **Weight (g)** | **Percent total pellet weight (%)** |
|---|---|---|
| **Sub-coated pellets** | | |
| Sugar Spheres (250-355µm) | 598.00 | 22.3 |
| Ethyl cellulose 7cps | 92.00 | 3.4 |
| Polyethylene glycol 400 | 17.25 | 0.6 |
| Dibutyl sebacate | 5.75 | 0.2 |

| **Drug layer** | | |
|---|---|---|
| Metoprolol succinate | 1092.50 | 40.9 |
| Polyvinyl pyrrolidone Povidone (PVP K-30) | 276.00 | 10.3 |

| **Controlled release film layer** | | |
|---|---|---|
| Ethyl cellulose 100cps | 473.80 | 17.7 |
| Polyethylene glycol 400 | 59.23 | 2.2 |
| Dibutyl sebacate | 59.23 | 2.2 |

| **Material** | **Weight (g)** | **Percent total weight (%)** |
|---|---|---|
| **Final blend and tableting** | | |
| Starlac | 3408.60 | 51.1 |
| Syloid 244 FP | 170.20 | 2.6 |
| Polyplasdone (Crospovidone XL 10) | 338.10 | 5.1 |
| Magnesium stearate | 80.50 | 1.2 |

In another embodiment the present invention provides a method of preparing a pharmaceutical composition comprising coated pellets comprising the steps of
a) providing an inert core comprising from about 50% to about 100% (w/w) of soluble substance;
b) applying a drug layer comprising the active pharmaceutical ingredient (API) onto the inert core forming a drug coated pellet;
c) coating the drug coated pellet with a controlled release layer forming a coated pellet.
A sub-coat layer covers an initial core/sphere forming the inert core. The initial core/sphere is preferably a sugar sphere and the amount of initial core/sphere is preferably from about 15% to about 25% by weight of the coated pellet. More preferably, the amount of initial core is about 22% of the coated pellet. In addition, the method preferably prepares a pharmaceutical composition comprising a plurality of coated pellets. The API (drug) is metoprolol or one of its pharmaceutical acceptable salts, each pellet thus comprising an inert core, a drug layer and a rate controlling film coating. Metoprolol succinate is the most preferred API. Moreover, the pharmaceutical composition is preferably prepared without the use of inherently toxic solvents.

The method of preparing a pharmaceutical composition of the present invention preferably further comprises sub-coating an initial core/sphere forming an inert core. Sub-coating an initial core/sphere comprises mixing a film coating polymer with one or more plasticizers in a solvent forming a coating mixture. Such mixture may be a solution, suspension or slurry for applying a coating layer on a surface. The coating mixture is applied to the initial core/sphere forming a sub-coated initial core/sphere which is used as an inert core in the present invention. The film coating polymer is a hydrophobic polymer. Suitable film coating polymers can be cellulose derivative polymers or polymethacrylate polymers, preferably ethylcellulose. The amount of ethylcellulose is preferably from about 75% to about 85% more preferably about 80% of the total amount of the weight of the sub-coat. Further, hydrophobic polymers or hydrophilic plasticizers, or a combination of several plasticizers can be used to plasticize the film coating polymers. These compounds of the polymeric sub-coat are mixed with solvents prior to their application onto the initial core/sphere. Suitable solvents for use in mixing the polymeric sub-coating compounds are selected from ethanol, isopropyl alcohol, acetone and purified water. A mixture of ethanol, acetone and water is preferred for use in mixing the polymeric sub-coating compounds.

Suitable plasticizers for use in sub-coating an initial core/sphere are selected from polyethylene glycol, dibutyl sebacate, and dibutyl phthalate. Preferred plasticizers are polyethylene glycol and dibutyl sebacate as the hydrophilic and hydrophobic plasticizers respectively. Preferred amounts of plasticizers used in the method are about 10% to about 20% polyethylene glycol and 3% to about 7% dibutyl sebacate by weight of the sub-coat. More preferably, about 15% polyethylene glycol and 5% dibutyl sebacate as plasticizer in the method of the present invention.

In the method of the present invention, preparing a pharmaceutical composition for extended release comprising coated pellets the particle size distribution of the drug substance is an important factor in binding the drug substance to the inert core. Preferably, the drug substance has a particle size distribution such that the d(0.9) value is less than about 80µm. More preferably, the d(0.9) value for the particle size distribution of the drug substance is less than about 50µm, most preferably less then about 30µm. To form a dispersion, the drug substance, a binder, and a solvent mixture are mixed to homogeneity. The solvent mixture comprises one or more of the solvents from the group, water, ethanol, acetone and isopropyl alcohol. Preferably, the solvent mixture is water. As a result, a thick or concentrated dispersion can be produced which may shorten the production time of applying the drug layer to the pellets. This dispersion of the drug substance, preferably a dispersion of metoprolol succinate, is then sprayed onto the inert core to form a drug coated pellet.

On these drug coated pellets a controlled release layer is applied in the method of the present invention. The compounds which make up the controlled release layer are mixed with solvents prior to their application onto the drug coated pellets to form a coating mixture. Suitable solvents for use in mixing the controlled release layer compounds are selected from ethanol, isopropyl alcohol, acetone and purified water, in order to achieve a high yield process, with a reasonable manufacturing time. A mixture of ethanol, acetone and water is preferred for use in mixing the controlled release layer compounds when these are a combination of ethyl cellulose, polyethylene glycol and dibutyl sebacate. The coating mixture is then sprayed onto the drug coated pellets forming controlled release drug coated pellets. This controlled release layer comprises water soluble and insoluble components. Such components may be film forming polymers and plasticizers. For example, a film comprising a polymeric layer is applied onto the drug coated pellets as the controlled release layer. The film comprises at least one film coating polymer and can be plasticized with one or more plasticizers. The controlled release layer in the pharmaceutical composition of the present invention preferably comprises a hydrophobic film coating polymer such as for example ethylcellulose and a combination of at least two plasticizers, at least one hydrophilic and one hydrophobic plasticizer. Preferably, the ratio of hydrophobic to hydrophilic plasticizer in the controlled release layer of the pharmaceutical composition of the present invention is from 3:1 to 1:3, more preferably the ratio is 1:1.

The method of the present invention may further comprise the steps of
d) mixing the coated pellets with a powder mixture of one or more excipients forming a final tableting blend;
e) pressing the final tableting blend into tablets; and
f) optionally film coating the tablets with a commercially available cosmetic tablet film coating.

Preferably, the final tableting blend in the method of the present invention is pressed into tablets using a direct compression procedure. In order to create a uniform blend for tableting, a particular ratio within the composition between the part of the coated pellets to the powder part is selected. The amount of coated pellets in the final tableting blend is preferably selected from about 20% to about 60% (by weight) in order to prepare such uniform product. More preferably, the amount of coated pellet in the final tableting blend is from about 30% to about 50% (by weight), most preferably from about 35% to about 45% (by weight).

In addition, the particle size distribution influences significantly the uniformity of the final blend and the final pharmaceutical product. The preferred uniformity of the tableting blend is such that the average assay of ten samples of the tableting blend each weighing the equivalent of one tablet lies within the range of 90 to 110 percent of the label dose and the relative standard deviation of the individual assays is less than or equal to 5 percent. To prepare such uniform product in the method of the present invention, preferably at least 50% (by weight) of the powder mixture has a particle size distribution between about 30µm to about 800µm, preferably from about 80µm to about 600µm, more preferably from about 100µm to about 300µm. More preferably, at least 65% (by weight) of the powder mixture has a particle size distribution between about 30µm to about 800µm, preferably from about 80µm to about 600µm, more preferably from about 100µm to about 300µm. Most preferably, at least 80% (by weight) of the powder mixture has a particle size distribution between about 30µm to about 800µm, preferably from about 80µm to about 600µm, most preferably from about 100µm to about 300µm.

In a preferred method of preparing a pharmaceutical composition of the present invention, the method comprises the following steps;
a) providing sugar spheres as initial cores;
b) coating the sugar spheres with a sub-coat comprising mixing a film of a hydrophobic polymer, a soluble (hydrophilic) plasticizer, and an insoluble (hydrophobic) plasticizer with a solvent mixture of e.g. acetone, ethanol 95%, and water and spraying the mixture onto the sugar spheres to create a sub-coat on the sugar spheres resulting in an inert core;
c) coating the sub-coated sugar spheres (inert cores) with a drug layer comprising mixing the drug, preferably metoprolol succinate, and a binder, preferably povidone (PVP K-30) with preferably water, forming an aqueous dispersion and applying the dispersion onto the sub-coated pellets (inert cores) forming drug coated pellets;
d) applying a third layer on the drug coated pellets comprising dissolving a hydrophobic film coating polymer, an hydrophilic plasticizer and an hydrophobic plasticizer in a solvent mixture of e.g. acetone, ethanol 95%, and water forming a mixture and spraying the mixture onto the drug coated pellets to create controlled release drug coated pellets;
e) mixing the controlled release drug coated pellets with a powder mixture of one or more excipients forming a final blend;
f) compressing the final blend into tablets or filling the final blend into capsules; and
g) optionally film coating the tablets for cosmetic purposes.

In this method of preparing a pharmaceutical composition the hydrophobic polymer is preferably ethyl cellulose (EC), the soluble/hydrophilic plasticizer is preferably polyethylene glycol (PEG), and the insoluble/hydrophobic plasticizer is preferably dibutyl sebacate (DBS). Further, in preparing a mixture for coating the sugar spheres with a sub-coat, and the drug coated pellets with a controlled release layer, ethyl cellulose is preferably first dissolved in acetone and ethanol 95%, then PEG and DBS are added, followed by adding water and mixing the solution till it is homogenized. Preferably, the spraying of a solution or dispersion onto sugar spheres or drug coated pellets in the method of the present invention uses a fluidized bed coater with a Wurster insertion. Furthermore, the binder, used in coating the sub-coated sugar spheres with a drug layer, facilitates binding of the drug to the inert core of sub-coated sugar spheres. Moreover, in this method of the present invention the ratio of powder mixture to controlled release drug coated pellets in the final tableting blend is preferably from about 20% to about 60% (by weight), more preferably from about 30% to about 50% (by weight), most preferably from about 35% to about 45% (by weight). As a result a uniform final tableting blend and tablets are produced.

In another embodiment the present invention also provides a method of treating patients with a beta₁-selective adrenoceptor blocking agent comprising administering to a patient in need thereof a pharmaceutical composition for extended release, comprising pellets coated with an active pharmaceutical ingredient wherein each coated pellet comprises; a) an inert core comprising from about 50% to about 100% (w/w) of soluble substance; b) a layer comprising metoprolol or a pharmaceutically acceptable salt thereof, which layer covers the inert core; and c) a controlled release layer thereon. Preferably, the method comprises treatment of patients suffering from hypertension, angina pectoris or stable symptomatic (NYHA Class II or III) heart failure of ischemic, hypertensive or cardiomyopathic origin.

The following examples are presented in order to further illustrate the invention.
These examples should not be construed in any manner to limit the invention.

### EXAMPLES

The following examples illustrate the parameters influencing the production of controlled release drug coated pellets for composition into the extended release pharmaceutical composition of the invention. The controlled release drug coated pellets preferably have a dissolution profile such that after 8 hours between about 20% and about 50% of the drug substance is dissolved when a sample of pellets equivalent to the desired dose is tested in the following conditions Method: Paddle @ 50rpm medium: 500ml 0.05M, Phosphate Buffer USP pH-6.8 at 37°C,

### Example 1 - Relationship between the release rate by initial inert core weight

The dissolution profile of a pharmaceutical composition can be altered by changing the amount of initial core used in the composition. A comparatively higher total weight of the initial core will result in a faster dissolution profile. In order to obtain a specific release rate for a given formulation the amount of a specific initial core required is carefully selected.

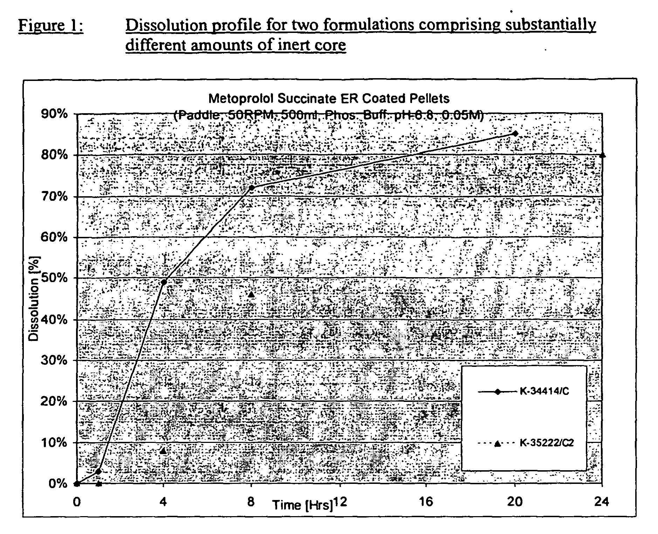

In table 1.1 data for two formulations that differ significantly in the amount of initial core weight are shown. In table 1.2 and in figure 1 in-vitro dissolution profiles for the two formulations are given where a plurality of pellets equivalent to 1 dose of 190mg Metoprolol succinate are dissolved using the parameters: Method: Paddle, 50rpm, 500ml 0.05M, Phosphate Buffer USP pH-6.8. These data show that the in-vitro dissolution profile is influenced by the amount of the initial core as a percentage of the final pellet that was used in each of the formulations.

**Table 1.1: Formulation ingredients and percentages**

| Function | Ingredient | K-34414/C (36.6% initial core) | | | K-35222/C2 (22.7% initial core) | | |
|---|---|---|---|---|---|---|---|
| | | [mg] | % of Function | % Coat w/w* | [mg] | % of Function | % Coat w/w* |
| **Initial core** | Sugar Spheres (250-355 µm) | **240** | 100% | NA** | **104** | 100% | NA** |
| Sub-Coat | Ethocel 7cps | 38.4 | 80% | 48mg 20% | 16 | 80% | 20mg 19.2% |
| | PEG 400 | 7.2 | 15% | | 3 | 15% | |
| | DBS | 2.4 | 5% | | 1 | 5% | |
| Drug Layer (20% Binder) | Metoprolol Succinate | 190 | 80% | 237.5mg 82.5% | 190 | 79.8% | 238mg 191.1% |
| | PVP K-30 | 47.5 | 20% | | 48 | 20.2% | |
| About 25% E.R. Coating | Ethocel 100cps | 105.2 | 80.0% | 131.5 mg 25.0% | 76.8 | 80.0% | 110mg 26.5% |
| | PEG 400 | 13.15 | 10.0% | | 9.6 | 10.0% | |
| | DBS | 13.15 | 10.0% | | 9.6 | 10.0% | |
| Total Weight | | 656.5 | | Fast | 458.0 | | Slow |

| | | | | | | | |
|---|---|---|---|---|---|---|---|
| * % Coat w/w refers to the percentage of the weight of the coating layer (i.e. sub-coat, drug layer, and E.R. coating) in comparison to the weight of the uncoated pellet (i.e. initial core, inert core (initial core and sub-coat), and drug layer pellets respectively). ** NA - Not applicable. | | | | | | | |

**Table 1.2: Dissolution Profile (amount of Metoprolol succinate released in %)**

| Time [Hrs] | K-34414/C | K-35222/C2 |
|---|---|---|
| 1 | 3% | 0% |
| 4 | 49% | 8% |
| 8 | 72% | 46% |
| 20 | 85% | NA |
| 24 | NA | 80% |

### Example 2 - Relationship between the release rate by the ratio of hydrophilic to hydrophobic plasticizers

The release rate from the coated pellets of the present invention is also affected by manipulating the ratio of the hydrophobic and hydrophilic components in the rate controlling layer. The preferred rate controlling layer in the present invention comprises ethyl cellulose (EC), an hydrophilic film coating polymer, and two types of plasticizers, dibutyl sebacate (DBS) and polyethylene glycol (PEG), an hydrophobic and an hydrophilic plasticizer, respectively. Changing the ratio of the EC and the plasticizer will change the release rate of the drug. In addition, changing the ratio between the two plasticizers will modify the in-vitro release rate (also known as dissolution profile) of the coated pellets.

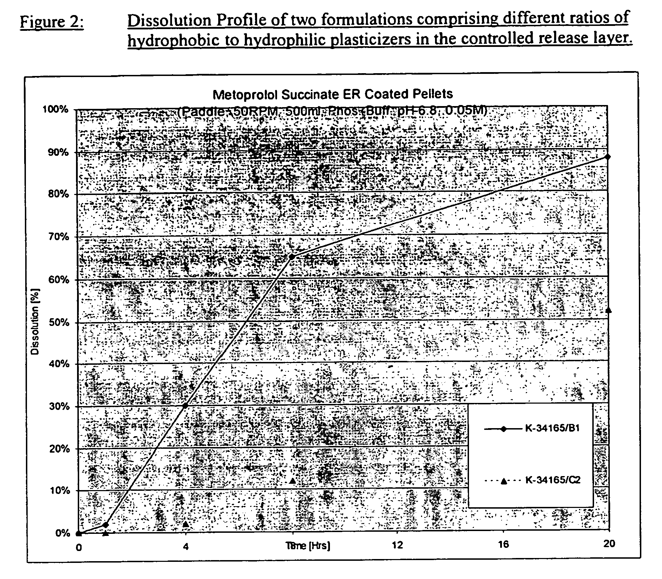

In table 2.1 data for 2 formulations that differ only in the ratio of the plasticizers in the controlled release layer coating is given. In table 2.2 and figure 2 in-vitro dissolution profiles for said two formulations is given using the dissolution method described above. The in vitro dissolution profile was strongly influenced by the ratio between the DBS and the PEG in the controlled release layer coating film.

**Table 2.1: Formulation ingredients and percentages**

| Function | Ingredient | K-34165/B1 | | | K-34165/C2 | | |
|---|---|---|---|---|---|---|---|
| | | [mg] | % in Fun. | % Coat w/w* | [mg] | % of Fun. | % Coat w/w* |
| Initial core | Cellets (200-355µm) | 290 | 100% | NA** | 290 | 100% | NA** |
| Drug Layer | Metoprolol Succinate | 190 | 84.8% | 224mg 77.2% | 190 | 84.8% | 224mg 77.2% |
| | PVP K-30 | 34 | 15.2% | | 34 | 15.2% | |
| **E.R. Coating** | Ethocel 100cps | 185.0 | 80.0% | 236.4mg 46% | 185.0 | 80.0% | 236.4mg 46% |
| | PEG 400 | 34.7 | **15.0%** | | 23.1 | **10.0%** | |
| | DBS | 11.6 | **5.0%** | | 23.1 | **10.0%** | |
| Total Weight | | 745.3 | | | 745.2 | | |

| | | | | | | | |
|---|---|---|---|---|---|---|---|
| * % Coat w/w refers to the percentage of the weight of the coating layer (i.e. drug layer, and E.R. coating) in comparison to the weight of the uncoated pellet (i.e. initial core, and drug layer pellets respectively). ** NA - Not applicable | | | | | | | |

**Table 2.2: Dissolution Profile (amount of Metoprolol succinate released in %)**

| Time [Hrs] | K-34165/B1 | K-34165/C2 |
|---|---|---|
| 1 | 2% | 0% |
| 4 | 30% | 2% |
| 8 | 65% | 12% |
| 20 | 88% | 52% |

### Example 3 - Retaining the integrity of the sugar spheres by sub coating the sugar spheres (initial cores), without changing the in vitro dissolution profile of the pellets.

In pellets compressed into a tablet drug product the pellets are mixed with a powder mixture that functions as glidant, filler, disintegrant, lubricant and cushioning agent. The pellets' size is usually larger than the size of the particles of the powder mixture, hence, the particles size distribution (PSD) of the blend of the pellets together with the powder mixture is wide. Such a wide PSD often tends to result in segregation and may cause a lack of uniformity in the final product, e.g.., the tablets or capsules. Moreover, high loading of drug on the pellets (per dose unit), will result in higher manifestation of this phenomenon.

In order to overcome this problem the drug is loaded onto inert core pellets which are relatively small in size. This may produce small sized pellets at the end of the process and the PSD of the overall final blend will thus be narrower. Commercially, there are a variety of pellets (initial cores) that can be coated (e.g. microcrystalline cellulose spheres, sugar spheres). When layering a large amount of drug on pellets having a small initial core, an initial core needs to be selected which can withstand a stressful process that may bring about attrition of the pellet core and even breaking of such pellet cores.

Such pellet cores can be strengthened by creating a film sub-coat, which preserves the integrity of the pellets' initial core. Such a film sub-coat may affect the release rate of the drug (also known as in-vitro dissolution profile), which may vary according to the type of such sub-coat. This phenomenon of fragility of the initial core is most pronounced when sugar spheres are used as the pellet initial core.

In the present invention a film sub-coat is applied to the initial core, which does not change the dissolution profile of the controlled release drug coated pellets. At the same time this sub-coat provides the required qualities which allows an extensive layering process to take place without attrition and breaking of the pellet initial core.

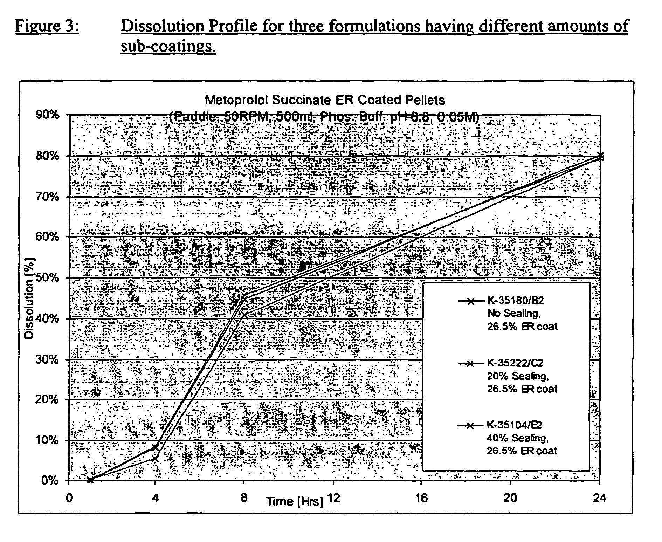

In table 3.1 data for three formulations that differ only in the percentage of the sub-coat is given. In table 3.2 and figure 3 in vitro dissolution profiles for said three formulations are given.

The in vitro dissolution profile was not influenced by the amount of the sub-coat that was applied on the pellet inert core.

**Table 3.1: Formulation ingredients and percentages**

| Function | Ingredient | K-35180/B2 | | | K-35222/C2 | | | K-35104/E2 | | |
|---|---|---|---|---|---|---|---|---|---|---|
| | | [mg] | % in Fun | % Coat w/w* | [mg] | % in Fun. | % Coat w/w | [mg] | % of Fun. | % Coat w/w* |
| Initial core | Sugar Spheres (250-355µm) | 104 | 100% | NA** | 104 | 100% | NA | 104 | 100% | NA** |
| **Sub-Coat** | Ethocel 7cps | NA** (0) | | | 16 | 80% | 20mg **19.2%** | 33.6 | 80% | 42mg **40.4%** |
| | PEG 400 | | | | 3 | 15% | | 6.3 | 15% | |
| | DBS | | | | 1 | 5% | | 2.1 | 5% | |
| Drug Layer (20%) Binder) | Metoprolol Succinate | 190 | 79.8% | 238mg 228.8% | 190 | 79.8% | 238mg 191.1% | 190 | 79.8% | 238mg 163.0% |
| | PVPK-30 | 48 | 20.2% | | 48 | 20.2% | | 48 | 20.2% | |
| 26.5% E.R. Coating | Ethocel 100cps | 72.6 | 80.0% | 90.8mg 26.5% | 76.8 | 80.0% | 96.0mg 26.5% | 81.5 | 80.0% | 101.9mg 26.5% |
| | PEG 400 | 9.1 | 10.0% | | 9.6 | 10.0% | | 10.2 | 10.0% | |
| | DBS | 9.1 | 10.0% | | 9.6 | 10.0% | | 10.2 | 10.0% | |
| Total Weight | | 432.8 | | | 458.0 | | | 485.9 | | |

| | | | | | | | | | | |
|---|---|---|---|---|---|---|---|---|---|---|
| * % Coat w/w refers to the percentage of the weight of the coating layer (i.e. sub-coat, drug layer, and E.R. coating) in comparison to the weight of the uncoated pellet (i.e. initial core, inert core (initial core and sub-coat), and drug layer pellets respectively). ** NA - Not Applicable | | | | | | | | | | |

**Table 3.2: Dissolution Profile (amount Metoprolol succinate released in %)**

| Time [Hrs] | K-35180/B2 No Sub-coat 26.5% E.R. Coat | K-35222/C2 20% Sub-coat 26.5% E.R. Coat | K-35104/E2 40% Sub-coat 26.5% E.R. Coat |
|---|---|---|---|
| 1 | 0% | 0% | 0% |
| 4 | 8% | 8% | 6% |
| 8 | 45% | 46% | 41% |
| 24 | 80% | 80% | 79% |

### Example 4 - The particle size distribution (PSD) of the Metoprolol Succinate

In order to apply a drug layer using a fluidized bed coater with a Wurster insertion (bottom spray process) a specific range of PSD for the active raw material should preferably be used.
In table 4.1 data for two experiments that differ only in the metoprolol succinate PSD is given. Also the assay results are given in table 4.1. The assay results indicate clearly that while working with metoprolol succinate with a higher d(0,9) value of d(0,9) NMT 80µm, the process produces lower assay results as compared to the example using metoprolol succinate with d(0.9) of NMT 25 µm, although just within acceptable limits. Hence, while spraying the metoprolol succinate as a dispersion in the layering process, the PSD of the active ingredient has a d(0,9) value of not more than 80 microns and preferably not more than 25 microns.

**Table 4.1: Formulation ingredients and PSD**

| Function | Ingredient | K-34803 | | K-34932 | |
|---|---|---|---|---|---|
| | | [mg]/PSD | | [mg]/PSD | |
| Initial core | Sugar Spheres (250-355µm) | 104 | | 104 | |
| Sub-Coat | Ethocel 7cps | 16 | | 16 | |
| | PEG 400 | 3 | | 3 | |
| | DBS | 1 | | 1 | |
| Drug Layer (20% Binder) | Metoprolol Succinate | 190 | d(0,9) NMT* 25 µm | 190 | d(0,9) NMT* 80µm |
| | PVP K-30 | 48 | NA** | 48 | NA** |
| Assay results | | 98.3% of the labeled amount | | 90.7% of the labeled amount | |

| | | | | | |
|---|---|---|---|---|---|
| * NMT - Not More Than ** NA - Not Applicable | | | | | |

### Example 5 - Use of solvents in the Sub-coat and contolled release coating process

In order to create the aforementioned sub-coat and controlled release coating films, a solution of EC, PEG and DBS was used in a bottom spray fluidized bed coater. In order to produce a reasonable process, with high yield, the solvents to be used should be carefully chosen. When the solvents used are not optimal a large percentage of the pellets agglomerate during the spraying and drying process.
In table 5.1 a few compositions of such solvent mixtures are given, as well as the amount of agglomerates formed, (determined by passing the coated pellets through a suitable screen e.g. 25 mesh). These agglomerates were eventually rejected from the batch. A mixture of acetone, alcohol and water for example should be carefully qualified when using the preferred mixture of EC, PEG and DBS in order to produce a high yield process.

**Table 5.1: Percentage composition of the solvents in Sub-coat & controlled release coating**

| | Batch Numbers | | | | | | |
|---|---|---|---|---|---|---|---|
| Ingredient | K3553/B | K35553/C | K35553/D | K35553/E | K35553/F | K35553/H | K35553/K |
| Ethanol 95% | 40% | 40% | 40% | 75% | 25% | 33% | 53% |
| Isopropyl Alcohol | 0% | 25% | 50% | 0% | 0% | 0% | 0% |
| Acetone | 50% | 25% | 0% | 25% | 75% | 67% | 33% |
| Water | 10% | 10% | 10% | 0% | 0% | 0% | 13% |
| %Agglomeration during controlled release coating (Rejected) | 5% | 34% | 44% | 73% | 46% | 14% | 6% |

As can be seen from the results in table 5.1, when using the preferred mixture of EC, PEG and DBS, the solvent mixture should comprise all of Ethanol 95%, Acetone and Water. It would appear that the use of about 10 % or more e.g. 13 % of water has a positive effect.

### Working Example 6 - Producing an extended release Metoprolol Succinate Tablet

The following batch may be produced after taking into account the considerations described in examples 1-5 although the amounts below are not to be taken as absolute but rather an exemplary composition of the formulations that can be manufactured.

**Table 6.1: Composition of a Metoprolol Succinate E.R. Tablet**

| Material | Weight [gr] per Batch | Note |
|---|---|---|
| **Sub-coated pellets (Inert Core) 26.6% w/w** | | |
| Sugar Spheres (250-355µm) | 598 | **(Initial Core)** |
| Ethyl cellulose 7cps | 92 | |
| Polyethylene glycol 400 | 17.25 | |
| Dibutyl sebacate | 5.75 | |
| Alcohol 95 % (Ethanol) | 345 | Process solvent |
| Acetone | 460 | Process solvent |
| Purified water | 115 | Process solvent |

| **Drug layer 51.2 w/w** | | |
|---|---|---|
| Metoprolol succinate | 1092.5 | PSD d(0,9) *NMT 30µm |
| Polyvinyl pyrrolidone Povidone (PVP K-30) | 276. | |
| Purified water | 2127.5 | Process solvent |

| **Controlled Release film layer 22.2 % w/w** | | |
|---|---|---|
| Ethyl cellulose 100cps | 473.8 | |
| Polyethylene glycol 400 | 59.23 | |
| Dibutyl sebacate | 59.23 | |
| Alcohol 95% (Ethanol) | 2760 | Process solvent |
| Acetone | 3450. | Process solvent |
| Purified water | 690. | Process solvent |

| **Final blend** | | |
|---|---|---|
| Starlac | 3408.6 | |
| Syloid 244 FP colloidal silicon dioxide | 170.2 | |
| Polyplasdone (Crospovidone XL 10) | 338.1 | |
| Magnesium stearate | 80.5 | |

| | | |
|---|---|---|
| * Not More Than | | |

### Preferred Manufacturing Process.

**Sub-coated pellets**: Add the ethyl cellulose to a mixture of acetone and alcohol , and mix for about 40 minutes until a clear solution is achieved. To that mixture add the polyethylene glycol 400 and dibutyl sebacate consecutively and stir the mixture for about ten minutes. Then, add purified water to the solution and stir for about twenty minutes. Spray the solution onto the sugar spheres (250-355µm) in a bottom spray fluidized bed coater (e.g. Glatt® GPCG 1.1), with an inlet temperature of about 45-50°C, and air flow of e.g. 30-60 m³/hr to create sub-coated pellets (Inert cores).
**Drug Coated Pellets**: Mix together purified water and polyvinyl pyrrolidone (PVP K-30) for about 20 minutes until homogeneity is obtained. Then, add metoprolol succinate and mix the dispersion for about 30 minutes before starting the process. Apply the drug dispersion onto the sub-coated pellets (inert cores) from the previous stage in a bottom spray fluidized bed coater (e.g. Glatt® GPCG 1.1), with an inlet temperature of about 55-65°C, and e.g. air flow of 30-60 m³/hr to create drug coated pellets.
**Controlled Release Drug Coated Pellets:** Add ethyl cellulose to a mixture of acetone and alcohol , and mix for about 40 minutes until a clear solution is achieved. To that mixture add polyethylene glycol 400 and dibutyl sebacate consecutively and stir the mixture for about ten minutes. Then, add purified water to the solution and stir for about twenty minutes. Spray the solution onto the drug coated pellets from the previous stage in a bottom spray fluidized bed coater (e.g. Glatt® GPCG 1.1), with an inlet temperature of about 45-50°C, and air flow of e.g. 30-60 m³/hr to create Controlled Release Drug Coated Pellets
**Final blend and Tableting or Capsule Filling:** Mix the Controlled Release Drug Coated Pellets with syloid and half of the starlac® quantity for 10 minutes using a dry blender (e.g. Twin Shelled "Y-cone" dry blender). Then, add the remaining quantity of starlac® and crospovidone to the dry blender, and mix for a further 15 minutes. Finally, add magnesium stearate, and mix for a further 5 minutes to produce final blend for tableting or capsule filling.
Check the final blend for uniformity of content by assay, and ensure that the results comply with the regulatory requirements of the e.g. current USP XXIX: average assay of ten samples each equivalent to the desired dose of between 90-110%, and RSD of not more than 5%.
The final blend may be compressed in a tableting machine e.g. Sivac® tablets compressing machine to create uniform tablets, as required by the USP, or filled into appropriately sized capsules.
Several strengths of metoprolol succinate E.R. tablets can be manufactured: e.g. 190mg, 95mg, 47.5mg and 23.75mg, which are equivalent to 200mg, 100mg, 50mg and 25mg of metoprolol tartrate respectively.

Tablets manufactured by a process as exemplified above were tested for rate of dissolution. The results, the dissolution profile for these tablets, are presented in table 6.2 below.

**Table 6.2: Dissolution Profile (amount Metoprolol succinate released in %)**

| Time [Hrs] | % dissolved from Tablets pressed from pellets produced as in Example 6 |
|---|---|
| 0 | 0% |
| 1 | 4% |
| 4 | 22 % |
| 8 | 48 % |
| 24 | 87 % |

In general tablets or capsules comprising pellets of the invention are acceptable when having the following dissolution profile:

| Time [Hrs] | % dissolved from Tablets or capsules comprising pellets produced by the process of the invention |
|---|---|
| 0 | 0% |
| 1 | Not More Than 25% |
| 4 | Between 10 and 40% |
| 8 | Between 30 and 60% |
| 24 | Not Less Than 70% |

### Example 7: Dissolution test

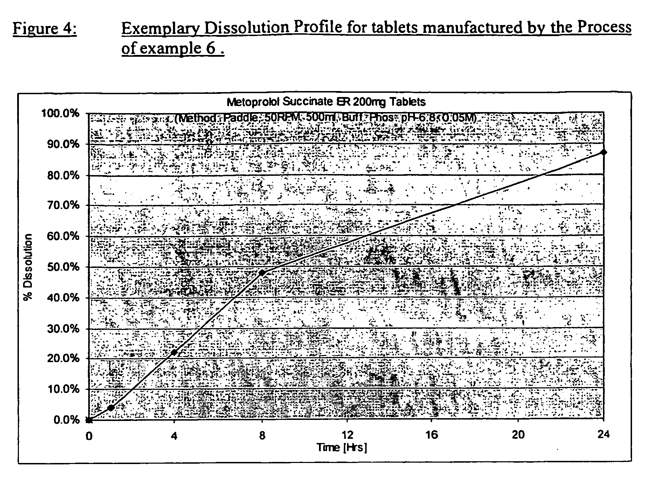

The pellets described in examples 1-3, and 6 were tested in a dissolution test wherein the pellets were dissolved in a media of 500ml of 0.05M phosphate buffer at a pH 6.8. The dissolution procedure was carried out in an USP Apparatus II, paddle method, at 37°C and 50 rpm. The amount of released metoprolol succinate was measured at 1, 4, 8, 20, and 24 hour time periods. The results are tabulated in the examples and graphically represented in figures 1 through 4.

## Claims

1. A pharmaceutical composition for extended release comprising pellets each coated with an active pharmaceutical ingredient wherein each coated pellet comprises
a) an inert core comprising at least 50% (w/w) of soluble substance;
b) a drug layer comprising metoprolol or a pharmaceutically acceptable salt thereof, which layer covers the inert core; and
c) a controlled release layer thereon;
and wherein the inert core comprises an initial core and a sub-coat thereon and wherein the sub-coat layer comprises at least one hydrophobic film coating polymer.

2. The pharmaceutical composition according to claim 1, wherein the pharmaceutical acceptable salt of metoprolol is metoprolol succinate.

3. The pharmaceutical composition according to claim 1 or claim 2, wherein the inert core comprises from about 70 weight% to about 90 weight% soluble substances.

4. The pharmaceutical composition according to any preceding claim, wherein the amount of inert core is from about 20% to about 35% by weight of each pellet.

5. The pharmaceutical composition according to claim 4, wherein the amount of inert core is about 27% by weight of each pellet.

6. The pharmaceutical composition according to claim 1, wherein the amount of initial core is from about 15% to about 25% by weight of each pellet.

7. The pharmaceutical composition according to claim 1, wherein the initial core is from about 15% to about 22% by weight of each pellet.

8. The pharmaceutical composition according to claim 1, wherein the amount of initial core is about 22% by weight of each pellet.

9. The pharmaceutical composition according to claim 1, wherein the initial core is a sugar sphere.

10. The pharmaceutical composition according to claim 1, wherein the size of the initial core is from about 50µm to about 500µm, preferably the size of the initial core is from about 100µm to about 400µm, more preferably the size of the initial core is from about 250µm to about 350µm.

11. The pharmaceutical composition according to claim 1, wherein the amount of the sub-coat is from about 10% to about 40% of the total weight of the sub-coated inert core, preferably the amount of the sub-coat is from about 15% to about 30% of the total weight of the sub-coated inert core, more preferably the amount of the sub-coat is about 16% of the total weight of the sub-coated inert core.

12. The pharmaceutical composition according to any one of the preceding claims, wherein the film coating polymer is selected from the group consisting of cellulose derivatives and/or polymethacrylates.

13. The pharmaceutical composition according to claim 12, wherein the film coating polymer is ethyl cellulose.

14. The pharmaceutical composition according to any one of the preceding claims, wherein the polymeric layer further comprises plasticizers selected from the group consisting of hydrophilic plasticizers, hydrophobic plasticizers, and mixtures thereof.

15. The pharmaceutical composition according to claim 14, wherein the plasticizers comprise at least one hydrophilic plasticizer and at least one hydrophobic plasticizer.

16. The pharmaceutical composition according to claim 15, wherein at least one hydrophobic plasticizers is selected from the group consisting of dibutyl sebacate and dibutyl phthalate.

17. The pharmaceutical composition according to claim 15 or claim 16, wherein at least one hydrophilic plasticizers is selected from the group consisting of triethyl citrate and polyethylene glycol.

18. The pharmaceutical composition according to any of the preceding claims, wherein the sub-coat comprises about 75% to about 85% ethyl cellulose, about 10% to about 20% polyethylene glycol, and about 3% to about 7% dibutyl sebacate by weight of the sub-coat.

19. The pharmaceutical composition according to claim 18, wherein the sub-coat comprises about 80% ethyl cellulose, about 15% polyethylene glycol, and about 5% dibutyl sebacate by weight of the sub-coat.

20. The pharmaceutical composition according to any preceding claim, wherein the drug layer comprises the active pharmaceutical ingredient and a binder.

21. The pharmaceutical composition according to claim 20, wherein the binder is selected from the group consisting of polyvinyl pyrrolidone (povidone), polymers of cellulose derivatives, and starch.

22. The pharmaceutical composition according to claim 21, wherein the binder is povidone.

23. The pharmaceutical composition according to any preceding claim, wherein the particle size distribution of the active pharmaceutical ingredient has a d(0.9) value of less than about 80µm.

24. The pharmaceutical composition according to claim 23, wherein the particle size distribution of the active pharmaceutical ingredient has a d(0.9) value of less than about 50µm, preferably the d(0.9) value is less than about 30µm, more preferably the d(0.9) value is about 2µm or less.

25. The pharmaceutical composition according to any preceding claim, wherein the amount of the drug layer is from about 40% to about 90% of the total weight of the combined inert core and drug layer, preferably the amount of the drug layer is from about 50% to about 80% of the total weight of the combined inert core and drug layer, more preferably the amount of the drug layer is from about 55% to about 75% of the total weight of the combined inert core and drug layer.

26. The pharmaceutical composition according to any preceding claim, wherein the controlled release layer comprises at least about 70% water insoluble compounds of the total weight of the controlled release layer, preferably the controlled release layer comprises at least about 80% water insoluble compounds of the total weight of the controlled release layer, more preferably the controlled release layer comprises at least about 90% water insoluble compounds of the total weight of the controlled release layer.

27. The pharmaceutical composition according to any preceding claim, wherein the controlled release layer is a film coating comprising a polymeric layer.

28. The pharmaceutical composition according to claim 27, wherein the polymeric layer comprises a hydrophobic film coating polymer and at least two plasticizers.

29. The pharmaceutical composition according to claim 28, wherein the hydrophobic film coating polymer is ethyl cellulose.

30. The pharmaceutical composition according to claim 28 or claim 29, wherein the at least two plasticizers are at least one hydrophilic plasticizer and one hydrophobic plasticizer.

31. The pharmaceutical composition according to claim 30, wherein the at least one hydrophilic plasticizer is selected from the group consisting of triethyl citrate and polyethylene glycol.

32. The pharmaceutical composition according to claim 30 or claim 31, wherein the at least one hydrophobic plasticizer is selected from the group consisting of dibutyl sebacate and dibutyl phthalate.

33. The pharmaceutical composition according to any of claims 30 to 32, wherein the ratio of the hydrophilic to hydrophobic plasticizers is 1:1.

34. The pharmaceutical composition according to any preceding claim, wherein the coated pellets have a size between 200µm and 800µm, preferably the coated pellets have a size between 300µm and 700µm, more preferably the coated pellets have a size between 400µm and 600µm.

35. The pharmaceutical composition according to any preceding claim, wherein the composition is in the form of a pharmaceutical dosage form.

36. The pharmaceutical composition according to claim 35, wherein the dosage form is selected from the group consisting of a tablet and a capsule.

37. The pharmaceutical composition according to claim 35 or claim 36, wherein the dosage form comprises a plurality of coated pellets and a powder mixture of one or more excipients.

38. The pharmaceutical composition according to claim 37, wherein at least 50% of the powder mixture has a particle size from about 30µm to about 800µm, preferably at least 50% of the powder mixture has a particle size from about 80µm to about 600µm, more preferably at least 50% of the powder mixture has a particle size from about 100µm to about 300µm.

39. The pharmaceutical composition according to claim 37, wherein at least 65% of the powder mixture has a particle size from about 30µm to about 800µm, preferably at least 65% of the powder mixture has a particle size from about 80pm to about 600µm, more preferably at least 65% of the powder mixture has a particle size from about 100µm to about 300µm.

40. The pharmaceutical composition according to claim 37, wherein at least 80% of the powder mixture has a particle size from about 30µm to about 800µm, preferably at least 80% of the powder mixture has a particle size from about 80µm to about 600µm, more preferably at least 80% of the powder mixture has a particle size from about 100µm to about 300µm.

41. The pharmaceutical composition according to any of claims 37 to 40, wherein the amount of coated pellets is from about 20% to about 60% of the weight of the dosage form, preferably the amount of coated pellets is from about 30% to about 50% of the weight of the dosage form, more preferably the amount of coated pellets is from about 35% to about 45% of the weight of the dosage form.

42. The pharmaceutical composition according to any of claims 37 to 41, wherein the excipients are selected from the group consisting of Starlac®, Cellactose®, Parteck®, Crospovidone, Silicon Dioxide, Magnesium Stearate, Talc, Zinc Stearate, Polyoxyethylene Stearate, Stearic Acid, and Cellulose derivatives.

43. The pharmaceutical composition according to any of claims 36 to 42, wherein the tablet further comprises a cosmetic film coat.

44. A pharmaceutical composition in tablet dosage form according to claim 36 comprising a plurality of pellets coated with an active pharmaceutical ingredient wherein each coated pellet comprises a) an initial core of sugar spheres coated with a plasticized film sub-coat of a hydrophobic film coating polymer plasticized with a hydrophilic and a hydrophobic plasticizer, b) a drug layer comprising a beta, specific adrenoceptor blocking agent and a binder, and c) a controlled release layer comprising a plasticized film coat of a hydrophobic film coating polymer plasticized with a hydrophilic and a hydrophobic plasticizer, and wherein the pellets are mixed with a final tableting blend comprising a powder mixture of two or more of fillers, disintegrants, glidants and lubricants, and wherein the hydrophobic film coating polymer comprises ethyl cellulose, the hydrophilic plasticizer comprises polyethylene glycol, the hydrophobic plasticizer comprises dibutyl sebacate, the beta₁ specific adrenoceptor blocking agent is metoprolol succinate, the binder comprises povidone, and the powder mixture comprises starlac, syloid, crospovidone and magnesium stearate.

45. A method of preparing a pharmaceutical composition comprising coated pellets according to any preceding claim comprising the steps of:
a) providing an inert core comprising at least 50% (w/w) of soluble substance;
b) applying a drug layer comprising metoprolol or a pharmaceutically acceptable salt thereof, onto the inert core forming a drug coated pellet;
c) coating the drug coated pellet with a controlled release layer;
and wherein the inert core comprises an initial core and a sub-coat thereon and wherein the sub-coat layer comprises at least one hydrophobic film coating polymer.

46. The method according to claim 45, wherein the pharmaceutically acceptable salt of metoprolol is metoprolol succinate.

47. The method according to claim 45, wherein the amount of the initial core is about 15% to 25% by weight of each pellet.

48. The method according to claim 45, wherein the amount of the initial core is about 15% to about 22% by weight of each pellet.

49. The method according to claim 45, wherein the amount of the initial core is about 22% by weight of each pellet.

50. The method according to claim 45, wherein the inert core comprises an initial core and a sub-coat and wherein the method further comprises coating the initial core with a sub-coat comprising the steps of:
a) mixing a film coating polymer with a soluble plasticizer and an insoluble plasticizer in a coating liquid forming a coating mixture; and
b) spraying the coating mixture onto the initial core/sphere.

51. The method according to claim 50, wherein the coating liquid is a mixture of one or more organic solvents and water.

52. The method according to claim 51, wherein the organic solvent is selected from the group consisting of ethanol, isopropyl alcohol, acetone, and mixtures thereof.

53. The method according to claim 51 or claim 52, wherein the organic solvent is a mixture of ethanol and acetone.

54. The method according to any of claims 45 to 53, wherein applying a drug layer comprising metoprolol or a pharmaceutically acceptable salt thereof onto the inert core forming a drug coated pellet comprises:
a) mixing the active pharmaceutical ingredient and a binder in a solvent mixture forming a dispersion; and
b) spraying the dispersion onto the inert core.

55. The method according to claim 54, wherein the binder is povidone.

56. The method according to claim 54 or claim 55, wherein the solvent mixture is water.

57. The method according to any of claims 45 to 56, wherein coating the drug coated pellet witch a controlled release layer comprises:
a) mixing a film coating polymer with a soluble plasticizer and an insoluble plasticizer in a coating liquid forming a mixture; and
b) spraying the mixture onto the drug coated pellet.

58. The method according to claim 57, wherein the coating liquid is a mixture of one or more organic solvents and water.

59. The method according to claim 58, wherein the organic solvent is selected from the group consisting of ethanol, isopropyl alcohol, acetone, and mixtures thereof.

60. The method according to claim 59, wherein the organic solvent is a mixture of ethanol and acetone.

61. The method according to any of claims 45 to 60 further comprising the steps of:
a) mixing the coated pellets with a powder mixture of one or more excipients forming a final blend;
b) pressing the final blend into tablets, or filling the final blend into capsules and
c) optionally film coating the tablets with a cosmetic tablet film coating.

62. The method according to claim 61, wherein pressing the final blend into tablets comprises direct compression of the final tableting blend.

63. The method according to claim 61, comprising preparing a pharmaceutical composition comprises the following steps:
a) providing sugar spheres as initial cores;
b) coating the sugar spheres with a sub-coat comprising mixing a film of a hydrophobic polymer, a soluble plasticizer, and an insoluble plasticizer with a solvent mixture of acetone, ethanol 95%, and water and spraying the mixture onto the sugar spheres to sub-coat the sugar spheres initial cores to create inert cores;
c) coating the sub-coated sugar spheres (inert cores) with a drug layer comprising mixing metoprolol succinate, and a binder, preferably povidone (PVP K-30) with water forming an aqueous dispersion and applying the dispersion on the sub-coated pellets forming drug coated pellets;
d) applying a third layer on the drug coated pellets comprising mixing a hydrophobic film coating polymer, an hydrophilic plasticizer and an hydrophobic plasticizer in a solvent mixture of acetone, ethanol 95%, and water forming a dispersion and spraying the mixture onto the drug coated pellets to create a controlled release layer on the drug coated pellets;
e) mixing the controlled release drug coated pellets with a powder mixture of one or more excipients forming a final blend and then compressed into tablets or filling into capsules; and
f) if pressed into tablets, optionally film coating the tablets with a cosmetic film coat.

64. Use of a pharmaceutical composition according to any of claims 1 to 44 for the manufacture of a medicament for the treatment of disorder susceptible to treatment with a beta₁-selective adrenoceptor blocking agent.

65. Use according to claim 64, wherein the disorder is selected from the group consisting of hypertension, angina pectoris and stable symptomatic (NYHA Class II or III) heart failure of ischemic, hypertensive or cardiomyopathic origin.

## Patentansprüche

1. Pharmazeutische Zusammensetzung für die Langzeitfreisetzung, umfassend Tabletten, die jeweils mit einem aktiven pharmazeutischen Bestandteil beschichtet sind, wobei jede beschichtete Tablette
a) einen inneren Kern mit wenigstens 50% (m/m) löslicher Substanz,
b) eine Arzneistoffschicht mit Metoprolol oder einem pharmazeutisch verträglichen Salz davon, wobei die Schicht den inneren Kern umgibt, und
c) eine Schicht für die kontrollierte Freisetzung darauf
umfasst, und wobei der innere Kern einen Initialkern und eine Unterbeschichtung darauf umfasst, und wobei die Unterbeschichtung wenigstens ein hydrophobes Filmbeschichtungspolymer umfasst.

2. Pharmazeutische Zusammensetzung nach Anspruch 1, wobei das pharmazeutisch verträgliche Salz von Metoprolol Metoprololsuccinat ist.

3. Pharmazeutische Zusammensetzung nach Anspruch 1 oder 2, wobei der innere Kern von etwa 70 Gew.-% bis etwa 90 Gew.-% lösliche Substanzen umfasst.

4. Pharmazeutische Zusammensetzung nach einem der vorangehenden Ansprüche, wobei die Masse des inneren Kerns von etwa 20% bis etwa 35% bezogen auf das Gewicht von jeder Tablette beträgt.

5. Pharmazeutische Zusammensetzung nach Anspruch 4, wobei die Masse des inneren Kerns etwa 27% bezogen auf das Gewicht jeder Tablette beträgt.

6. Pharmazeutische Zusammensetzung nach Anspruch 1, wobei die Masse des inneren Kerns von etwa 15% bis etwa 25% bezogen auf das Gewicht von jeder Tablette beträgt.

7. Pharmazeutische Zusammensetzung nach Anspruch 1, wobei der innere Kern von etwa 15% bis etwa 22% bezogen auf das Gewicht von jeder Tablette ausmacht.

8. Pharmazeutische Zusammensetzung nach Anspruch 1, wobei die Masse des inneren Kerns etwa 22% bezogen auf das Gewicht von jeder Tablette ausmacht.

9. Pharmazeutische Zusammensetzung nach Anspruch 1, wobei der innere Kern eine Zuckerkugel ist.

10. Pharmazeutische Zusammensetzung nach Anspruch 1, wobei die Größe des inneren Kerns von etwa 50 µm bis etwa 500 µm beträgt, vorzugsweise beträgt die Größe des inneren Kerns von etwa 100 µm bis etwa 400 µm, noch bevorzugter beträgt die Größe des inneren Kerns von etwa 250 µm bis etwa 350 µm.

11. Pharmazeutische Zusammensetzung nach Anspruch 1, wobei die Masse der Unterbeschichtung von etwa 10% bis etwa 40% des Gesamtgewichts des mit der Unterbeschichtung überzogenen inneren Kerns beträgt, vorzugsweise beträgt die Masse der Unterbeschichtung von etwa 15% bis etwa 30% des Gesamtgewichts des mit der Unterbeschichtung überzogenen inneren Kerns, noch bevorzugter beträgt die Masse der Unterbeschichtung etwa 16% des Gesamtgewichts des mit der Unterbeschichtung überzogenen inneren Kerns.

12. Pharmazeutische Zusammensetzung nach einem der vorangehenden Ansprüche, wobei das Filmbeschichtungspolymer aus der Gruppe ausgewählt ist, die aus Zellulosederivaten und/oder Polymethacrylaten besteht.

13. Pharmazeutische Zusammensetzung nach Anspruch 12, wobei das Filmbeschichtungspolymer Ethylzellulose ist.

14. Pharmazeutische Zusammensetzung nach einem der vorangehenden Ansprüche, wobei die Polymerschicht weiterhin Weichmacher umfasst, die ausgewählt sind aus der Gruppe, bestehend aus hydrophilen Weichmachern, hydrophoben Weichmachern und Gemischen davon.

15. Pharmazeutische Zusammensetzung nach Anspruch 14, wobei die Weichmacher wenigstens einen hydrophilen Weichmacher und wenigstens einen hydrophoben Weichmacher umfassen.

16. Pharmazeutische Zusammensetzung nach Anspruch 15, wobei wenigstens ein hydrophober Weichmacher aus der Gruppe ausgewählt ist, die aus Dibutylsebacat und Dibutylphthalat besteht.

17. Pharmazeutische Zusammensetzung nach Anspruch 15 oder Anspruch 16, wobei wenigstens ein hydrophiler Weichmacher aus der Gruppe ausgewählt ist, die aus Triethylcitrat und Polyethylenglycol besteht.

18. Pharmazeutische Zusammensetzung nach einem der vorangehenden Ansprüche, wobei die Unterbeschichtung etwa 75% bis etwa 85% Ethylzellulose, etwa 10% bis etwa 20% Polyethylenglycol und etwa 3% bis etwa 7% Dibutylsebacat bezogen auf das Gewicht der Unterbeschichtung umfasst.

19. Pharmazeutische Zusammensetzung nach Anspruch 18, wobei die Unterbeschichtung etwa 80% Ethylzellulose, etwa 15% Polyethylenglycol und etwa 5% Dibutylsebacat bezogen auf das Gewicht der Unterbeschichtung umfasst.

20. Pharmazeutische Zusammensetzung nach einem der vorangehenden Ansprüche, wobei die Arzneistoffschicht den aktiven pharmazeutischen Bestandteil und ein Bindemittel umfasst.

21. Pharmazeutische Zusammensetzung nach Anspruch 20, wobei das Bindemittel aus der Gruppe ausgewählt ist, bestehend aus Polyvinylpyrrolidon (Povidon), Polymeren von Zellulosederivaten und Stärke.

22. Pharmazeutische Zusammensetzung nach Anspruch 21, wobei das Bindemittel Povidon ist.

23. Pharmazeutische Zusammensetzung nach einem der vorangehenden Ansprüche, wobei die Partikelgrößenverteilung des aktiven pharmazeutischen Bestandteils einen d(0.9)-Wert von weniger als etwa 80 µm aufweist.

24. Pharmazeutische Zusammensetzung nach Anspruch 23, wobei die Partikelgrößenverteilung des aktiven pharmazeutischen Bestandteils einen d(0.9)-Wert von weniger als etwa 50 µm aufweist, vorzugsweise beträgt der d(0.9)-Wert weniger als etwa 30 µm, noch bevorzugter beträgt der d(0.9)-Wert etwa 25 µm oder weniger.

25. Pharmazeutische Zusammensetzung nach einem der vorangehenden Ansprüche, wobei die Menge der Arzneistoffschicht von etwa 40% bis etwa 90% des Gesamtgewichts der Kombination von innerem Kern und Arzneistoffschicht beträgt, vorzugsweise ist die Menge der Arzneistoffschicht von etwa 50% bis etwa 80% des Gesamtgewichts der Kombination von innerem Kern und Arzneistoffschicht, noch bevorzugter ist die Menge der Arzneistoffschicht von etwa 55% bis etwa 75% des Gesamtgewichts der Kombination von innerem Kern und Arzneistoffschicht.

26. Pharmazeutische Zusammensetzung nach einem der vorangehenden Ansprüche, wobei die Schicht für die kontrollierte Freisetzung wenigstens etwa 70% wasser-unlösliche Verbindungen an dem Gesamtgewicht der Schicht für die kontrollierte Freisetzung umfasst, vorzugsweise umfasst die Schicht für die kontrollierte Freisetzung wenigstens etwa 80% wasser-unlösliche Verbindungen des Gesamtgewichts der Schicht für die kontrollierte Freisetzung, noch bevorzugter umfasst die Schicht für die kontrollierte Freisetzung wenigstens etwa 90% wasser-unlösliche Verbindungen des Gesamtgewichts der Schicht für die kontrollierte Freisetzung.

27. Pharmazeutische Zusammensetzung nach einem der vorangehenden Ansprüche, wobei die Schicht für die kontrollierte Freisetzung eine Filmbeschichtung ist, die eine Polymerschicht umfasst.

28. Pharmazeutische Zusammensetzung nach Anspruch 27, wobei die Polymerschicht ein hydrophobes Filmbeschichtungspolymer und wenigstens zwei Weichmacher umfasst.

29. Pharmazeutische Zusammensetzung nach Anspruch 28, wobei das hydrophobe Filmbeschichtungspolymer Ethylzellulose ist.

30. Pharmazeutische Zusammensetzung nach Anspruch 28 oder 29, wobei die wenigstens zwei Weichmacher wenigstens ein hydrophiler Weichmacher und ein hydrophober Weichmacher sind.

31. Pharmazeutische Zusammensetzung nach Anspruch 30, wobei der wenigstens eine hydrophile Weichmacher aus der Gruppe ausgewählt ist, die aus Triethylcitrat und Polyethylenglycol besteht.

32. Pharmazeutische Zusammensetzung nach Anspruch 30 oder 31, wobei der wenigstens eine hydrophobe Weichmacher ausgewählt ist aus der Gruppe, die aus Dibutylsebacat und Dibutylphthalat besteht.

33. Pharmazeutische Zusammensetzung nach einem der Ansprüche 30 bis 32, wobei das Verhältnis von hydrophilen zu hydrophoben Weichmachern 1:1 beträgt.

34. Pharmazeutische Zusammensetzung nach einem der vorangehenden Ansprüche, wobei die beschichteten Tabletten eine Größe zwischen 200 µm und 800 µm aufweisen, vorzugsweise haben die beschichteten Tabletten eine Größe zwischen 300 µm und 700 µm, noch bevorzugter haben die beschichteten Tabletten eine Größe zwischen 400 µm und 600 µm.

35. Pharmazeutische Zusammensetzung nach einem der vorangehenden Ansprüche, wobei die Zusammensetzung in der Form einer pharmazeutischen Dosierungsform vorliegt.

36. Pharmazeutische Zusammensetzung nach Anspruch 35, wobei die Dosierungsform aus der Gruppe ausgewählt ist, die aus einer Tablette und einer Kapsel besteht.

37. Pharmazeutische Zusammensetzung nach Anspruch 35 oder Anspruch 36, wobei die Dosierungsform eine Mehrzahl von beschichteten Tabletten umfasst und ein Pulvergemisch von einem oder mehreren Hilfsstoffen.

38. Pharmazeutische Zusammensetzung nach Anspruch 37, wobei wenigstens 50% des Pulvergemisches eine Partikelgröße von etwa 30 µm bis etwa 800 µm aufweist, vorzugsweise haben wenigstens 50% des Pulvergemisches eine Partikelgröße von etwa 80 µm bis etwa 600 µm, noch bevorzugter haben wenigstens 50% des Pulvergemisches eine Partikelgröße von etwa 100 µm bis etwa 300 µm.

39. Pharmazeutische Zusammensetzung nach Anspruch 37, wobei wenigstens 65% des Pulvergemisches eine Partikelgröße von etwa 30 µm bis etwa 800 µm aufweisen, vorzugsweise haben wenigstens 65% des Pulvergemisches eine Partikelgröße von etwa 80 µm bis etwa 600 µm, noch bevorzugter haben wenigstens 65 % des Pulvergemisches eine Partikelgröße von etwa 100 µm bis etwa 300 µm.

40. Pharmazeutische Zusammensetzung nach Anspruch 37, wobei wenigstens 80% des Pulvergemisches eine Partikelgröße von etwa 30 µm bis etwa 800 µm aufweisen, vorzugsweise haben wenigstens 80% des Pulvergemisches eine Partikelgröße von etwa 80 µm bis etwa 600 µm, noch bevorzugter haben wenigstens 80% des Pulvergemisches eine Partikelgröße von etwa 100 µm bis etwa 300 µm.

41. Pharmazeutische Zusammensetzung nach einem der Ansprüche 37 bis 40, wobei die Masse der beschichteten Tabletten von etwa 20% bis etwa 60% des Gewichts der Dosierungsform ausmacht, vorzugsweise beträgt die Masse der beschichteten Tabletten von etwa 30% bis etwa 50% des Gewichts der Dosierungsform, noch bevorzugter beträgt die Masse der beschichteten Tabletten von etwa 35% bis etwa 45% des Gewichts der Dosierungsform.

42. Pharmazeutische Zusammensetzung nach einem der Ansprüche 37 bis 41, wobei die Hilfsstoffe ausgewählt sind aus der Gruppe, bestehend aus Starlac®, Cellactose®, Parteck®, Crospovidon, Siliciumdioxid, Magnesiumstearat, Talcum, Zinkstearat, Polyoxyethylenstearat, Stearinsäure und Zellulosederivaten.

43. Pharmazeutische Zusammensetzung nach einem der Ansprüche 36 bis 42, wobei die Tablette außerdem eine kosmetische Filmbeschichtung umfasst.

44. Pharmazeutische Zusammensetzung in Tablettendosierungsform nach Anspruch 36, umfassend eine Mehrzahl an Tabletten, die mit einem aktiven pharmazeutischen Bestandteil beschichtet sind, wobei jede beschichtete Tablette a) einen Initialkern aus Zuckerkugeln, die mit einer Weichfilm-Unterbeschichtung eines mit einem hydrophilen und einem hydrophoben Weichmacher plastifizierten hydrophoben Filmbeschichtungspolymers beschichtet sind, b) eine Arzneistoffschicht mit einem Beta₁-spezifischen Adrenorezeptorblocker und einem Bindemittel und c) eine Schicht für die kontrollierte Freisetzung, umfassend eine Weichfilm-Schicht eines mit einem hydrophilen und einem hydrophoben Weichmacher plastifizierten hydrophoben Filmbeschichtungspolymers umfasst, und wobei die Tabletten mit einem Fertigtablettengemisch, welches ein Pulvergemisch von zwei oder mehr Füllstoffen, Zerfallsstoffen, Gleitstoffen und Schmierstoffen umfasst, vermischt werden, und wobei das hydrophobe Filmbeschichtungspolymer Ethylzellulose umfasst, der hydrophile Weichmacher Polyethylenglycol umfasst, der hydrophobe Weichmacher Dibutylsebacat umfasst, der Beta₁-spezifische Adrenorezeptorblocker Metoprololsuccinat ist, das Bindemittel Povidon umfasst und das Pulvergemisch Starlac, Syloid, Crospovidon und Magnesiumstearat umfasst.

45. Verfahren zur Herstellung einer pharmazeutischen Zusammensetzung, die beschichtete Tabletten umfasst, nach einem der vorangehenden Ansprüche mit den Stufen:
a) Bereitstellen eines inneren Kerns mit wenigstens 50% (m/m) an löslicher Substanz,
b) Aufbringen einer Arzneistoffschicht, umfassend Metoprolol oder ein pharmazeutisch verträgliches Salz davon, auf den inneren Kern unter Ausbildung einer arzneistoffbeschichteten Tablette,
c) Beschichten der arzneistoffbeschichteten Tablette mit einer Schicht für die kontrollierte Freisetzung,
und wobei der innere Kern einen Initialkern und eine Unterbeschichtung darauf umfasst, und wobei die Unterbeschichtungsschicht wenigstens ein hydrophobes Filmbeschichtungspolymer umfasst.

46. Verfahren nach Anspruch 45, wobei das pharmazeutisch verträgliche Salz von Metoprolol Metoprololsuccinat ist.

47. Verfahren nach Anspruch 45, wobei die Masse des Initialkerns etwa 15% bis 25% bezogen auf das Gewicht von jeder Tablette beträgt.

48. Verfahren nach Anspruch 45, wobei die Masse des Initialkerns etwa 15% bis etwa 22% bezogen das Gewicht von jeder Tablette beträgt.

49. Verfahren nach Anspruch 45, wobei die Masse des Initialkerns etwa 22% bezogen auf das Gewicht von jeder Tablette beträgt.

50. Verfahren nach Anspruch 45, wobei der innere Kern einen Initialkern und eine Unterbeschichtung aufweist, und wobei das Verfahren weiterhin umfasst, dass man den Initialkern mit einer Unterbeschichtung beschichtet, wobei man:
a) ein Filmbeschichtungspolymer mit einem löslichen Weichmacher und einem unlöslichen Weichmacher in einer Beschichtungsflüssigkeit unter Ausbildung eines Beschichtungsgemisches vermischt, und
b) das Beschichtungsgemisch auf den Initialkern bzw. die initiale Kugel sprüht.

51. Verfahren nach Anspruch 50, wobei die Beschichtungsflüssigkeit ein Gemisch von einem oder mehreren organischen Lösungsmitteln und Wasser ist.

52. Verfahren nach Anspruch 52, wobei das organische Lösungsmittel aus der Gruppe ausgewählt ist, bestehend aus Ethanol, Isopropylalkohol, Aceton und Gemischen davon.

53. Verfahren nach Anspruch 51 oder Anspruch 52, wobei das organische Lösungsmittel ein Gemisch von Ethanol und Aceton ist.

54. Verfahren nach einem der Ansprüche 45 bis 53, wobei das Aufbringen einer Arzneistoffschicht mit Metoprolol oder einem pharmazeutisch verträglichen Salz davon auf den inneren Kern unter Ausbildung einer arzneistoffbeschichteten Tablette umfasst, dass man:
a) den aktiven pharmazeutischen Bestandteil und ein Bindemittel in einem Lösungsmittelgemisch unter Ausbildung einer Dispersion vermischt, und
b) die Dispersion auf den inneren Kern sprüht.

55. Verfahren nach Anspruch 54, wobei das Bindemittel Povidon ist.

56. Verfahren nach Anspruch 54 oder Anspruch 55, wobei das Lösungsmittelgemisch Wasser ist.

57. Verfahren nach einem der Ansprüche 45 bis 56, wobei das Beschichten der arzneistoffbeschichteten Tablette mit einer Schicht für die kontrollierte Freisetzung umfasst, dass man:
a) ein Filmbeschichtungspolymer mit einem löslichen Weichmacher und einem unlöslichen Weichmacher in einer Beschichtungsflüssigkeit unter Ausbildung eines Gemisches vermischt, und
b) das Gemisch auf die arzneistoffbeschichtete Tablette sprüht.

58. Verfahren nach Anspruch 57, wobei die Beschichtungsflüssigkeit ein Gemisch von einem oder mehreren organischen Lösungsmitteln und Wasser ist.

59. Verfahren nach Anspruch 58, wobei das organische Lösungsmittel aus der Gruppe ausgewählt ist, bestehend aus Ethanol, Isopropylalkohol, Aceton und Gemischen davon.

60. Verfahren nach Anspruch 59, wobei das organische Lösungsmittel ein Gemisch von Ethanol und Aceton ist.

61. Verfahren nach einem der Ansprüche 45 bis 60, welches weiterhin die Stufen umfasst:
a) Mischen der beschichteten Tabletten mit einem Pulvergemisch von einem oder mehreren Hilfsstoffen unter Ausbildung eines Fertiggemisches,
b) Pressen des Fertiggemisches in Tabletten oder Füllen des Fertiggemisches in Kapseln, und
c) wahlweise Filmbeschichten der Tabletten mit einer kosmetischen Tablettenfilmbeschichtung.

62. Verfahren nach Anspruch 61, wobei das Pressen des Fertiggemisches in Tabletten das Direktpressen des fertigen Tablettiergemisches umfasst.

63. Verfahren nach Anspruch 61, welches die Herstellung einer pharmazeutischen Zusammensetzung umfasst mit den folgenden Stufen:
a) Bereitstellen von Zuckerkugeln als Initialkerne,
b) Beschichten der Zuckerkugeln mit einer Unterbeschichtung, umfassend das Mischen eines Films eines hydrophoben Polymers, eines löslichen Weichmachers und eines unlöslichen Weichmachers mit einem Lösungsmittelgemisch von Aceton, Ethanol 95% und Wasser, und Sprühen des Gemisches auf die Zuckerkugeln, um die Zuckerkugel-Initialkerne mit einer Unterbeschichtung zu versehen, um inerte Kerne zu erzeugen.
c) Beschichten der mit einer Unterbeschichtung versehenen Zuckerkugeln (inerte Kerne) mit einer Arzneistoffschicht, umfassend ein Gemisch von Metoprololsuccinat und einem Bindemittel, vorzugsweise Povidon (PVP K-30), mit Wasser unter Ausbildung einer wässrigen Dispersion und Aufbringen der Dispersion auf die mit einer Unterbeschichtung versehenen Tabletten unter Ausbildung von arzneistoffbeschichteten Tabletten,
d) Aufbringen einer dritten Schicht auf die Arzneistoffbeschichteten Tabletten, umfassend das Vermischen eines hydrophoben Filmbeschichtungspolymers, eines hydrophilen Weichmachers und eines hydrophoben Weichmachers in einem Lösungsmittelgemisch von Aceton, Ethanol 95% und Wasser unter Ausbildung einer Dispersion und Sprühen des Gemisches auf die arzneistoffbeschichteten Tabletten, um eine Schicht für die kontrollierte Freisetzung auf den arzneistoffbeschichteten Tabletten auszubilden,
e) Mischen der für die kontrollierte Arzneistofffreisetzung beschichteten Tabletten mit einem Pulvergemisch von einem oder mehreren Hilfsstoffen unter Ausbildung eines Fertiggemisches, und dann Pressen in Tabletten oder Abfüllen in Kapseln, und
f) wahlweise Filmbeschichtung der Tabletten mit einer kosmetischen Filmbeschichtung, im Falle des Pressens in Tabletten.

64. Verwendung einer pharmazeutischen Zusammensetzung nach einem der Ansprüche 1 bis 44 für die Herstellung eines Arzneimittels für die Behandlung von Störungen, die empfänglich sind für die Behandlung mit einem Beta₁-selektiven Adrenorezeptorblocker.

65. Verwendung nach Anspruch 64, wobei die Störung ausgewählt ist aus der Gruppe bestehend aus Bluthochdruck, Angina pectoris und Herzfehler ischämischen, hypertensiven oder kardiomyopathischen Ursprungs mit stabiler Symptomatik (NYHA-Klasse II oder III).

## Revendications

1. Composition pharmaceutique à libération prolongée comprenant des granulés revêtus respectivement d'un ingrédient pharmaceutique actif, chaque comprimé revêtu comprenant
a) un noyau inerte comprenant au moins 50 % (p/p) de substance soluble ;
b) une couche de médicament comprenant du métoprolol ou un sel pharmaceutiquement acceptable de celui-ci, ladite couche couvrant le noyau inerte ; et
c) une couche à libération contrôlée sur celle-ci ;
et dans laquelle le noyau inerte comprend un noyau initial et un sous-revêtement sur celui-ci et la couche de sous-revêtement comprenant au moins un polymère de revêtement en film hydrophobe.

2. Composition pharmaceutique selon la revendication 1, dans laquelle le sel de métoprolol pharmaceutiquement acceptable est le succinate de métoprolol.

3. Composition pharmaceutique selon la revendication 1 ou la revendication 2, dans laquelle le noyau inerte comprend d'environ 70 % en poids à environ 90 % en poids de substances solubles.

4. Composition pharmaceutique selon l'une quelconque des revendications précédentes, dans laquelle la quantité de noyau inerte est d'environ 20 % à environ 35 % en poids de chaque granulé.

5. Composition pharmaceutique selon la revendication 4, dans laquelle la quantité de noyau inerte est d'environ 27 % en poids de chaque granulé.

6. Composition pharmaceutique selon la revendication 1, dans laquelle la quantité de noyau initial est d'environ 15 % à environ 25 % en poids de chaque granulé.

7. Composition pharmaceutique selon la revendication 1, dans laquelle le noyau initial est d'environ 15 % à environ 22 % en poids de chaque granulé.

8. Composition pharmaceutique selon la revendication 1, dans laquelle la quantité de noyau initial est d'environ 22 % en poids de chaque granulé.

9. Composition pharmaceutique selon la revendication 1, dans laquelle le noyau initial est une sphère de sucre.

10. Composition pharmaceutique selon la revendication 1, dans laquelle la taille du noyau initial est d'environ 50 µm à environ 500 µm, de préférence la taille du noyau initial est d'environ 100 µm à environ 400 µm, de manière davantage préférée, la taille du noyau initial est d'environ 250 µm à environ 350 µm.

11. Composition pharmaceutique selon la revendication 1, dans laquelle la quantité de sous-revêtement est d'environ 10 % à environ 40 % du poids total du noyau inerte sous-revêtu, de préférence, la quantité du sous-revêtement est d'environ 15 % à environ 30 % du poids total du noyau inerte sous-revêtu, de manière davantage préférée, la quantité du sous-revêtement est d'environ 16 % du poids total du noyau inerte sous-revêtu.

12. Composition pharmaceutique selon l'une quelconque des revendications précédentes, dans laquelle le polymère de revêtement en film est choisi dans le groupe comprenant les dérivés de cellulose et/ou les polyméthacrylates.

13. Composition pharmaceutique selon la revendication 12, dans laquelle le polymère de revêtement en film est l'éthylcellulose.

14. Composition pharmaceutique selon l'une quelconque des revendications précédentes, dans laquelle la couche polymère comprend en outre des plastifiants choisis dans le groupe comprenant des plastifiants hydrophiles, des plastifiants hydrophobes et leurs mélanges.

15. Composition pharmaceutique selon la revendication 14, dans laquelle les plastifiants comprennent au moins un plastifiant hydrophile et au moins un plastifiant hydrophobe.

16. Composition pharmaceutique selon la revendication 15, dans laquelle au moins un plastifiant hydrophobe est choisi dans le groupe comprenant le dibutylsébaçate et le dubytlphtalate.

17. Composition pharmaceutique selon la revendication 15 ou la revendication 16, dans laquelle au moins un plastifiant hydrophile est choisi dans le groupe comprenant le triéthylcitrate et le polyéthylène glycol.

18. Composition pharmaceutique selon l'une quelconque des revendications précédentes, dans laquelle le sous-revêtement comprend environ 75 % à environ 85 % d'éthylcellulose, environ 10 % à environ 20 % de polyéthylène glycol et d'environ 3 % à environ 7 % de dibutylsébaçate en poids du sous-revêtement.

19. Composition pharmaceutique selon la revendication 18, dans laquelle le sous-revêtement comprend environ 80 % d'éthylcellulose, environ 15 % de polyéthylène glycol et environ 5 % de dibutylsébaçate en poids du sous-revêtement.

20. Composition pharmaceutique selon l'une quelconque des revendications précédentes, dans laquelle la couche de médicament comprend l'ingrédient pharmaceutique actif et un liant.

21. Composition pharmaceutique selon la revendication 20, dans laquelle le liant est choisi dans le groupe comprenant la polyvinylpyrrolidone (povidone), les polymères de dérivés de cellulose et l'amidon.

22. Composition pharmaceutique selon la revendication 21, dans laquelle le liant est la povidone.

23. Composition pharmaceutique selon l'une quelconque des revendications précédentes, dans laquelle la distribution des tailles de particules de l'ingrédient pharmaceutique actif a une valeur d(0,9) inférieure à environ 80 µm.

24. Composition pharmaceutique selon la revendication 23, dans laquelle la distribution des tailles de particules de l'ingrédient pharmaceutique actif a une valeur d(0,9) inférieure à environ 50 µm, de préférence la valeur d(0,9) est inférieure à environ 30 µm, de manière davantage préférée, la valeur d(0,9) est d'environ 25 µm ou inférieure.

25. Composition pharmaceutique selon l'une quelconque des revendications précédentes, dans laquelle la quantité de couche médicamenteuse est d'environ 40 % à environ 90 % du poids total du noyau inerte et de la couche de médicament combinés, de préférence, la quantité de la couche de médicament est d'environ 50 % à environ 80 % du poids total du noyau inerte et de la couche de médicament combinés, de manière davantage préférée, la quantité de la couche de médicament est d'environ 55 % à environ 75 % du poids total du noyau inerte et de la couche de médicament combinés.

26. Composition pharmaceutique selon l'une quelconque des revendications précédentes, dans laquelle la couche à libération contrôlée comprend au moins environ 70 % de composés insolubles dans l'eau du poids total de la couche à libération contrôlée, de préférence la couche à libération contrôlée comprend au moins environ 80 % de composés insolubles dans l'eau du poids total de la couche à libération contrôlée, de manière davantage préférée, la couche à libération contrôlée comprend au moins environ 90 % de composés insolubles dans l'eau du poids total de la couche à libération contrôlée.

27. Composition pharmaceutique selon l'une quelconque des revendications précédentes, dans laquelle la couche à libération contrôlée est un revêtement en film comprenant une couche polymère.

28. Composition pharmaceutique selon la revendication 27, dans laquelle la couche polymère comprend un polymère de revêtement en film hydrophobe et au moins deux plastifiants.

29. Composition pharmaceutique selon la revendication 28, dans laquelle le polymère de revêtement en film hydrophobe est l'éthylcellulose.

30. Composition pharmaceutique selon la revendication 28 ou la revendication 29, dans laquelle au moins les deux plastifiants sont au moins un plastifiant hydrophile et un plastifiant hydrophobe.

31. Composition pharmaceutique selon la revendication 30, dans laquelle au moins un plastifiant hydrophile est choisi dans le groupe comprenant le triéthylcitrate et le polyéthylène glycol.

32. Composition pharmaceutique selon la revendication 30 ou la revendication 31, dans laquelle au moins un plastifiant hydrophobe est choisi dans le groupe comprenant le dibutyl-sébaçate et le dibutylphtalate.

33. Composition pharmaceutique selon l'une quelconque des revendications 30 à 32, dans laquelle le rapport des plastifiants hydrophiles aux plastifiants hydrophobes est de 1:1.

34. Composition pharmaceutique selon l'une quelconque des revendications précédentes, dans laquelle les granulés revêtus ont une taille entre 200 µm et 800 µm, de préférence, les granulés revêtus ont une taille entre 300 µm et 700 µm, de manière davantage préférée, les granulés revêtus ont une taille entre 400 µm et 600 µm.

35. Composition pharmaceutique selon l'une quelconque des revendications précédentes, la composition étant sous la forme d'une forme galénique pharmaceutique.

36. Composition pharmaceutique selon la revendication 35, dans laquelle la forme galénique est choisie dans le groupe comprenant un comprimé et une capsule.

37. Composition pharmaceutique selon la revendication 35 ou la revendication 36, dans laquelle la forme galénique comprend une pluralité de granulés revêtus et un mélange en poudre d'un ou plusieurs excipients.

38. Composition pharmaceutique selon la revendication 37, dans laquelle au moins 50 % du mélange en poudre a une taille de particules d'environ 30 µm à environ 800 µm, de préférence au moins 50 % du mélange en poudre ont une taille de particules d'environ 80 µm à environ 600 µm, de manière davantage préférée, au moins 50 % du mélange en poudre ont une taille de particules d'environ 100 µm à environ 300 µm.

39. Composition pharmaceutique selon la revendication 37, dans laquelle au moins 65 % du mélange en poudre ont une taille de particules d'environ 30 µm à environ 800 µm, de préférence au moins 65 % du mélange en poudre ont une taille de particules d'environ 80 µm à environ 600 µm, de manière davantage préférée, au moins 65 % du mélange en poudre ont une taille de particules d'environ 100 µm à environ 300 µm.

40. Composition pharmaceutique selon la revendication 37, dans laquelle au moins 80 % du mélange en poudre ont une taille de particules d'environ 30 µm à environ 800 µm, de préférence au moins 80 % du mélange en poudre ont une taille de particules d'environ 80 µm à environ 600 µm, de manière davantage préférée, au moins 80 % du mélange en poudre ont une taille de particules d'environ 100 µm à environ 300 µm.

41. Composition pharmaceutique selon l'une quelconque des revendications 37 à 40, dans laquelle la quantité de granulés revêtus est d'environ 20 % à environ 60 % du poids de la forme galénique, de préférence la quantité des granulés revêtus est d'environ 30 % à environ 50 % du poids de la forme galénique, de manière davantage préférée, la quantité des granulés revêtus est d'environ 35 % à environ 45 % du poids de la forme galénique.

42. Composition pharmaceutique selon l'une quelconque des revendications 37 à 41, dans laquelle les excipients sont choisis dans le groupe comprenant le Starlac®, le Cellactose®, le Parteck®, la crospovidone, le dioxyde de silicium, le stéarate de magnésium, le talc, le stéarate de zinc, le stéarate de polyoxyéthylène, l'acide stéarique et les dérivés de cellulose.

43. Composition pharmaceutique selon l'une quelconque des revendications 36 à 42, dans laquelle le comprimé comprend en outre un revêtement en film cosmétique.

44. Composition pharmaceutique sous forme de comprimé selon la revendication 36, comprenant une pluralité de granulés revêtus d'un ingrédient pharmaceutique actif, dans laquelle chaque granulé revêtu comprend a) un noyau initial de sphères de sucre revêtu d'un sous-revêtement en film plastifié d'un polymère de revêtement en film hydrophobe plastifié avec un plastifiant hydrophile et un plastifiant hydrophobe, b) une couche de médicament comprenant un agent inhibiteur de l'adréno-récepteur bêtal-spécifique et un agent liant, et c) une couche à libération contrôlée comprenant un revêtement en film plastifié d'un polymère de revêtement en film hydrophobe plastifié avec un plastifiant hydrophile et un plastifiant hydrophobe, et dans laquelle les granulés sont mélangés avec un mélange de pastillage final comprenant un mélange en poudre de deux composants ou plus parmi les agents de charge, les agents de désintégration, les agents glissants et les lubrifiants, et dans laquelle le polymère de revêtement en film hydrophobe comprend l'éthylcellulose, le plastifiant hydrophile comprend le polyéthylène glycol, le plastifiant hydrophobe comprend le dibutylsébaçate, l'agent inhibiteur de l'adrénorécepteur bêtal-spécifique est le succinate de métoprolol, l'agent liant comprend la povidone et le mélange en poudre comprend le starlac, le syloïde, la crospovidone et le stéarate de magnésium.

45. Procédé de préparation d'une composition pharmaceutique comprenant des granulés revêtus selon l'une quelconque des revendications précédentes, comprenant les étapes consistant à :
a) fournir un noyau inerte comprenant au moins 50 % (p/p) de substance soluble ;
b) appliquer une couche de médicament comprenant le métoprolol ou un sel pharmaceutiquement acceptable de celui-ci, sur le noyau inerte formant un granulé revêtu de médicament ;
c) revêtir le granulé revêtu de médicament avec un couche à libération contrôlée ; et dans lequel le noyau inerte comprend un noyau initial et un sous-revêtement sur celui-ci et dans lequel la couche de sous-revêtement comprend au moins un polymère de revêtement en film hydrophobe.

46. Procédé selon la revendication 45, dans lequel le sel de métoprolol pharmaceutiquement acceptable est le succinate de métoprolol.

47. Procédé selon la revendication 45, dans lequel la quantité de noyau initial est d'environ 15 % à 25 % en poids de chaque granulé.

48. Procédé selon la revendication 45, dans lequel la quantité du noyau initial est d'environ 15 % à environ 22 % en poids de chaque comprimé.

49. Procédé selon la revendication 45, dans lequel la quantité de noyau initial est d'environ 22 % en poids de chaque granulé.

50. Procédé selon la revendication 45, dans lequel le noyau inerte comprend un noyau initial et un sous-revêtement et dans lequel le procédé comprend en outre le revêtement du noyau initial avec un sous-revêtement comprenant les étapes consistant à :
a) mélanger un polymère de revêtement en film avec un plastifiant soluble et un plastifiant insoluble dans un liquide de revêtement formant un mélange de revêtement ; et
b) pulvériser le mélange de revêtement sur le noyau/sphère initial(e).

51. Procédé selon la revendication 50, dans lequel le liquide de revêtement est un mélange d'un ou plusieurs solvants organiques et d'eau.

52. Procédé selon la revendication 51, dans lequel le solvant organique est choisi dans le groupe comprenant l'éthanol, l'alcool d'isopropyle, l'acétone et leurs mélanges.

53. Procédé selon la revendication 51 ou la revendication 52, dans lequel le solvant organique est un mélange d'éthanol et d'acétone.

54. Procédé selon l'une quelconque des revendications 45 à 53, dans lequel l'application d'une couche de médicament comprenant le métoprolol ou un sel pharmaceutiquement acceptable de celui-ci sur le noyau inerte formant un granulé revêtu de médicament comprend :
a) le mélange de l'ingrédient pharmaceutique actif et d'un liant dans un mélange de solvants formant une dispersion ; et
b) la pulvérisation de la dispersion sur le noyau inerte.

55. Procédé selon la revendication 54, dans lequel le liant est la povidone.

56. Procédé selon la revendication 54 ou la revendication 55, dans lequel le mélange de solvant est de l'eau.

57. Procédé selon l'une quelconque des revendications 45 à 56, dans lequel le revêtement des granulés revêtus de médicament avec une couche à libération contrôlée comprend :
a) le mélange d'un polymère de revêtement en film avec un plastifiant soluble et d'un plastifiant insoluble dans un liquide de revêtement formant un mélange ; et
b) la pulvérisation du mélange sur les granulés revêtus de médicament.

58. Procédé selon la revendication 57, dans lequel le liquide de revêtement est un mélange d'un ou plusieurs solvants organiques et d'eau.

59. Procédé selon la revendication 58, dans lequel le solvant organique est choisi dans le groupe comprenant l'éthanol, l'alcool d'isopropyle, l'acétone et leurs mélanges.

60. Procédé selon la revendication 59, dans lequel le solvant organique est un mélange d'éthanol et d'acétone.

61. Procédé selon l'une quelconque des revendications 45 à 60, comprenant en outre les étapes consistant à :
a) mélanger les granulés revêtus avec un mélange en poudre d'un ou plusieurs excipients formant un mélange final ;
b) la compression du mélange final en comprimés ou le conditionnement du mélange final dans des capsules et
c) éventuellement, le revêtement en film des comprimés avec un revêtement en film de comprimé cosmétique.

62. Procédé selon la revendication 61, dans lequel la compression du mélange final en comprimés comprend la compression directe du mélange de pastillage final.

63. Procédé selon la revendication 61, comprenant la préparation d'une composition pharmaceutique comprenant les étapes suivantes :
a) la fourniture de sphères de sucre comme noyaux initiaux ;
b) le revêtement des sphères de sucre avec un sous-revêtement comprenant le mélange d'un film d'un polymère hydrophobe, d'un plastifiant soluble et d'un plastifiant insoluble avec un mélange de solvants d'acétone, d'éthanol à 95 % et d'eau et la pulvérisation du mélange sur les sphères de sucre pour effectuer un sous-revêtement des noyaux initiaux des sphères de sucre pour créer des noyaux inertes ;
c) le revêtement des sphères de sucre sous-revêtues (noyaux inertes) avec une couche de médicament comprenant le mélange du succinate de métoprolol et un liant, de préférence la povidone (PVP K-30) avec de l'eau formant une dispersion aqueuse et l'application de la dispersion sur les granulés sous-revêtus formant des granulés revêtus de médicament ;
d) l'application d'une troisième couche sur les granulés revêtus de médicament comprenant le mélange d'un polymère de revêtement en film hydrophobe, d'un plastifiant hydrophile et d'un plastifiant hydrophobe dans un mélange de solvants d'acétone, d'éthanol à 95 % et d'eau formant une dispersion et la pulvérisation du mélange sur les granulés revêtus de médicament pour créer une couche à libération contrôlée sur les granulés revêtus de médicament ;
e) le mélange des granulés revêtus de médicament à libération contrôlée avec un mélange en poudre d'un ou plusieurs excipients formant un mélange final puis compressés en comprimés ou conditionnés dans des capsules ; et
f) dans le cas de la compression en comprimés, éventuellement, le revêtement en film des comprimés avec un revêtement en film cosmétique.

64. Utilisation d'une composition pharmaceutique selon l'une quelconque des revendications 1 à 44, pour la production d'un médicament destiné au traitement de troubles sensibles au traitement avec un agent inhibiteur de l'adrénorécepteur bêta1-sélectif

65. Utilisation selon la revendication 64, dans laquelle le trouble est choisi dans le groupe comprenant l'hypertension, l'angine de poitrine et l'insuffisance cardiaque symptomatique stable (NYHA de classe II ou III) d'origine ischémique, hypertensive ou cardiomyopathique.
